# EUROPEAN PATENT SPECIFICATION

(11) **EP 4 168 798 B1**
(45) Date of publication and mention of the grant of the patent: **06.08.2025**
(21) Application number: 21724743.6
(22) Date of filing: 29.04.2021
(51) Int. Cl.: G01N 33/487

(54) **NANOPORE SUPPORT STRUCTURE AND MANUFACTURE THEREOF**
NANOPORENTRÄGERSTRUKTUR UND HERSTELLUNG DAVON
STRUCTURE DE SUPPORT DE NANOPORE ET SA FABRICATION

(30) Priority: 17.06.2020 US 202063040455 P; 23.10.2020 GB 202016874; 23.04.2021 GB 202105787
(43) Date of publication of application: 26.04.2023
(73) Proprietor: Oxford Nanopore Technologies plc, Oxford Science Park Oxford OX4 4DQ (GB)
(72) Inventor: XIE, Ping, Oxford Oxfordshire OX4 4DQ (GB); MILLIS, Justin, Oxford Oxfordshire OX4 4DQ (GB); HEALY, Ken, Oxford Oxfordshire OX4 4DQ (GB); CLARKE, James Anthony, Oxford Oxfordshire OX4 4DQ (GB); HYDE, Jason Robert, Oxford Oxfordshire OX4 4DQ (GB); WILTSHIRE, Richard Kenneth John, Oxford Oxfordshire OX4 4DQ (GB); MCKENDRY, Jonathan Edward, Oxford Oxfordshire OX4 4DQ (GB); GREASTY, Robert, Oxford Oxfordshire OX4 4DQ (GB); BROWN, Clive Gavin, Oxford Oxfordshire OX4 4DQ (GB); PERA, Ioana, Oxford Oxfordshire OX4 4DQ (GB); SANGHERA, Gurdial Singh, Oxford Oxfordshire OX4 4DQ (GB); HYLAND, Mark, Oxford Oxfordshire OX4 4DQ (GB); ORTIZ BAHAMON, Pedro Miguel, Oxford Oxfordshire OX4 4DQ (GB); JACKSON, Mark David, Oxford Oxfordshire OX4 4DQ (GB); MACKETT, Paul Raymond, Oxford Oxfordshire OX4 4DQ (GB); DAVIES, Rhodri Rhys, Oxford Oxfordshire OX4 4DQ (GB)
(74) Representative: EIP
(86) International application number: PCT/GB2021/051029
(87) International publication number: WO 2021/255414

(56) References cited:
- GB-A- 2 574 048
- GB-A- 2 574 048
- JP-A- 2013 118 852
- JP-A- 2013 118 852
- US-A1- 2005 070 042
- US-A1- 2005 070 042
- US-A1- 2006 231 419
- US-A1- 2006 231 419
- US-A1- 2015 265 994
- US-A1- 2015 265 994
- US-A1- 2017 059 546
- US-A1- 2017 059 546
- US-A1- 2017 168 040
- US-A1- 2017 168 040
- KANG XINQI ET AL: "One-Pot Species Release and Nanopore Detection in a Voltage-Stable Lipid Bilayer Platform", NANO LETTERS, vol. 19, no. 12, 11 December 2019 (2019-12-11), US, pages 9145 - 9153, XP055910095, ISSN: 1530-6984, DOI: 10.1021/acs.nanolett.9b04446

## Description

The present invention relates to nanopore support structures.

Nanopore sensors have been developed for sensing a wide range of species, including single molecules such as polymer molecules. A known nanopore sensor device is a MinION^{™}, manufactured and sold by Oxford Nanopore Technologies Ltd. The nanopore-based sensing therein employs the measurement of ionic current flow through a biological nanopore located in a highly resistive amphiphilic membrane. The MinION^{™} has an array of nanopore sensors. As a molecule, such as a polymer analyte e.g. DNA, is caused to translocate a nanopore, measurement of the fluctuations in ionic current may be used to determine the sequence of the DNA strand. Nanopore devices for detection of analytes other than polynucleotides such as proteins are also known from WO2013/123379.

The amphiphilic membrane in which the nanopore is located is typically supported on a support structure. Many designs of such structures are known. For example, US2015/265994, discloses designs of supports for an array of membranes. US2015/265994 discloses nanopore support structures comprising inner portions defining inner recesses, which function as wells, without gaps between them and outer portions that look like pillars that extend outwardly from the inner portions and have gaps therebetween. In such a nanopore support structure, the outer portions (pillars) are outside the footprint of the inner recesses (wells) of the inner portions. Supported membranes extend across the openings of the inner recesses. Thus, the actual membrane is relatively large, which creates a large membrane capacitance and makes the membrane mechanically less robust. However, if the inner recess is reduced in size, then the volume of the reservoir of fluid in the inner recess is reduced and therefore exhausted more quickly. Thus, the size of the inner recesses is a balance between the membrane properties and the volume of available fluid in the inner recess.

Not only are known nanopore support structures e.g. disclosed in US2015/265994, a balance between the membrane properties and the volume of available fluid in the inner recess, but they have been found to have an influence on the membrane, wherein using this type of nanopore support structure where an access hole is opened at the bottom of each well, the membrane is mobile across the whole support structure. With the extra pressure caused by membrane surface tension, the solution inside the well has a tendency to drain through the access hole slowly and the membrane will eventually collapse.

Reliable formation of membranes with desirable properties is always a difficulty. It would be advantageous to provide improved support structures that promote the formation of membranes that are more stable, and have properties better suited to the sensing applications for which they are to be used.

The present invention is concerned with such improved nanopore support structures,.

US-2017/0059546 discloses a nanopore support structure comprising: a dielectric layer comprising walls defining a plurality of wells; and hydrophilic and hydrophobic layers above the dielectric layer, the hydrophilic and hydrophobic layers forming overhangs extending from the walls across each of the wells, the overhang defining an aperture configured to support a lipid bilayer suitable for insertion of a nanopore lipid at the interface between the hydrophobic and hydrophilic layers.

According to a first aspect of the present invention, there is provided a nanopore support structure according to independent claim 1.

Including overhangs defines an aperture across which the membrane can be formed. This allows the well size to be made larger while reducing the actual membrane size and area. **In** turn, this reduces the capacitance of the membrane, and improves the stability of the membrane. The aperture size can also be chosen independently of the well size, providing greater flexibility in the design parameters of the nanopore support structure. The well size may be increased in depth and/or cross-sectional area. Increasing the well size allows more buffer solution to be stored in the well, allowing a nanopore sensing device including such a nanopore support structure to operate for longer periods of time before the buffer becomes depleted.

The protrusions protruding laterally of the extent of the overhangs allow the surface and structural properties of the overhangs to be further controlled, thereby improving the performance of the nanopore support structure and the sensing device in which it functions.

The inner protrusions protruding laterally of the extent of the overhang inside the respective wells provide control of the surface and structural properties of the overhangs both inside the well.

In some embodiments, the walls can be provided without features that retain apolar medium, e.g. oil, such as indentations or projections. The walls can be featureless. The walls can be substantially smooth. Provision of the walls without apolar medium retaining features has the advantage that wells having electrodes on their walls or at their respective bases may be provided of a greater depth and aspect ratio without ingress of apolar medium onto electrodes in the well. This enables a larger well volume and a greater amount of a redox electrode mediator to be provided in the well, enabling a longer lifetime of the device in operation.

In other embodiments, the walls may be provided with apolar medium control features such as projections. Such apolar medium control features which may extend across the entirety of the walls or a part of the walls extending from the overhangs.

In some embodiments, the protrusions include outer protrusions protruding laterally of the extent of the overhang outside the respective wells. The outer protrusions provide control of the surface and structural properties of the overhangs outside the well.

In some embodiments, the protrusions are arranged to increase retention of apolar medium by the overhangs. Retention of apolar medium on the surface of overhangs of the nanopore support structure promotes formation of the membrane on the nanopore support structure. Alternatively or additionally, in some embodiments the nanopore support structure comprises outer protrusions protruding laterally of the extent of an outer surface of the wall layer between the wells. Some outer protrusions may extend from both the outer surface of the wall layer and from one or more overhangs.

In some embodiments, for each aperture, the outer protrusions define a respective protrusion wall at least partially surrounding and set back from the aperture. Providing a protrusion wall may enable a meniscus to be formed across the respective aperture such that the meniscus extends, at least in part, into the respective aperture. This greater control of the meniscus may be used to assist formation of membranes in the aperture, and may avoid the need for pre-treatment to promote membrane formation.

In some embodiments the nanopore support structure comprises, for each aperture, a plurality of peripheral outer protrusions comprising an inner surface facing towards the aperture that defines the protrusion wall, and an outer surface facing away from the aperture. The inner and/or outer surface of one or more of the peripheral outer protrusions may comprise micropatterned structures.

In some embodiments, the outer protrusions further comprise intermediate outer protrusions arranged in an area between the peripheral outer protrusions. The intermediate outer protrusions may help deliver and control an apolar medium used in formation of membranes across the apertures. One or more intermediate outer protrusions may comprise micropattered structures. In particular, one or more surfaces of one or more intermediate outer protrusions may comprise micropatterned structures.

In some embodiments, the protrusions are arranged to increase rigidity of the overhangs. This reduces the chance of damage, thereby improving lifetime of the structure and improving the consistency of the performance of the device.

In some embodiments, the wells have respective bases. The bases of the wells may be used to support further structures that are important in a nanopore sensing device, such as electrodes or further apertures.

In some embodiments, the wall layer further defines the bases. This may be convenient in some applications, as the base layer can be formed as part of the same process that defines the wall layers.

In some embodiments, the nanopore support structure further comprises a substrate, the wall layer being fixed to the substrate and the substrate defining the bases of the wells. Using a separate substrate to define the bases may be advantageous depending on the manufacturing method, and where it is desirable to use a different material to define the base from that used for the wall layer.

In some embodiments, the overhangs and the wall layer comprise cured, negative photoresist material. This is advantageous because the photoresist material can be formed into the desired structures through controlled exposure of the resist to light.

In some embodiments, the nanopore support structure further comprises barriers disposed in the wells, the barriers being capable of reducing scattering of electromagnetic radiation of a wavelength for curing the negative photoresist material. Scattering can cause exposure of the photoresist material in unwanted regions, thereby changing the structure of the nanopore support structure from its intended design. Barriers reduce this effect by reducing scattering of light.

In some embodiments in which the wells have respective bases, the barriers extend from the bases to the overhangs. This improves the scattering reduction provided by the barriers, and allows them to also provide structural support to the overhangs in some embodiments.

In some embodiments, the barriers extend from the walls inwardly into the wells, and optionally the barriers are curved along their extent inwardly into the wells. This can improve the ability of the barriers to provide structural support, particularly in some embodiments of the manufacturing methods.

In some embodiments, the wall layer and the overhangs are respective moulded components that are fixed together.

In some embodiments, the nanopore support structure further comprises membranes extending across respective apertures and optionally also nanopores inserted in at least some of the membranes.

According to a second aspect of the present invention, there is provided a nanopore sensing device comprising first and second chambers, a planar structure comprising a nanopore support structure according to the first aspect of the present invention, the planar structure being provided with plural fluidic passages which extend between the first and second chambers and include respective wells and apertures of said nanopore support structure, the apertures opening into the first chamber, and electrodes arranged to sense a fluidic electrical potential in respective passages between the nanopores and the second chamber.

The nanopore sensing device may comprise an array of membranes, each membrane provided across a respective aperture. Each membrane may comprise a nanopore.

As discussed above, the nanopore support structures of the first aspect of the invention provide advantages in terms of their structural and electrical properties. A nanopore sensing device comprising these nanopore support structures will therefore have improved reliability and flexibility of its design. For example, the nanopore support structures contribute to balancing the pressure across the membrane and pining the membrane to the aperture location. This makes it compatible with wells with openings or through holes at the bottom that can be used to provide the passages between the nanopores and the second chamber.

In some embodiments, the passages can comprise fluidic resistor portions between the electrode and the second chamber. These fluidic resistor portions can create a fluidic resistance between the electrode and second chamber comparable to the resistance through the nanopore between the electrode and the first chamber. This permits advantageous configurations for the measurement of the fluidic electrical potential.

In some embodiments, the planar structure comprises a further layer, the fluidic resistor portions being formed in the further layer. Placing the fluidic resistor portions in a further layer permits additional design flexibility relative to requiring them to be formed by the wall layer or substrate of the nanopore support structure.

In some embodiments, the first and second chambers are on opposite sides of the planar structure and the passages extend through the planar structure. This permits the flow of ion through the nanopore to allow measurements of the properties of molecules passing through the nanopore.

In some embodiments, the nanopore sensing device further comprises drive electrodes in the first and second chambers. These permit the application of voltage to drive an ion current between the two chambers and allow the measurement of the properties of molecules passing through the nanopore.

To allow better understanding, embodiments of the present invention will now be described by way of non-limitative example with reference to the accompanying drawings, in which:
Fig. 1a is a cross-sectional side view of a nanopore support structure, Fig. 1b is a plan view of an array of the nanopore support structures in Fig. 1a, and Fig. 1c illustrates a bilayer in a nanopore support structure;
Fig. 2 is a cross-sectional side view of a nanopore support structure having an alternate form;
Fig. 3 is a cross-sectional side view of a nanopore sensing device incorporating a nanopore support structure;
Fig. 4 is a cross-sectional side view of an overhang of a well having protrusions;
Figs. 5a to 5e are cross-sectional side views of the nanopore support structure during steps of a first method of manufacture using photoresist;
Figs. 6a to 6c are side views of the nanopore support structure during steps of a second method of manufacture using photoresist and Fig. 6d is a side view of the resultant nanopore support structure having a membrane formed therein;
Fig. 7a is a side view of a comparative example of a nanopore support structure without an overhang and Fig. 7b is a side view of the nanopore support structure made using the second method of manufacture having a membrane formed therein;
Figs. 8a and 8b are side views of the nanopore support structure during steps of a third method of manufacture using photoresist;
Figs. 9a and 9b are side views of the nanopore support structure during steps of a fourth method of manufacture using photoresist and Fig. 9c is a plan view of the resultant nanopore support structure;
Figs. 10a to 10l are side views of the nanopore support structure during steps of a fourth method of manufacture not in accordance with the claimed invention, using moulding;
Fig. 11 is a graph of open pore current against time for a conventional nanopore support structure, and a nanopore support structure;
Fig. 12 shows possible shapes of apertures in the nanopore support structures.
Fig. 13 is a schematic cross-sectional representation of a sensing device, or component thereof, having a turreted well array structure connected to a substrate having electrodes at the base of the walls;
Figs. 14(a) to 14(f) are cross-sectional views of process steps not in accordance with the claimed invention, that can be used to make the component of Figure 13;
Fig. 15 is a perspective view of a mould having a protrusion with shortened fins;
Fig. 16 is a perspective view a single compartment of an improved well array structure not in accordance with the claimed invention, in which recesses are provided at the base of the compartment;
Figs. 17(a) to 17(e) are schematic cross-sectional representations of method steps for forming a sensing device, or component thereof, having a turreted well array structure connected to a substrate that is coated, drilled, filled and etched before the well array structure is connected;
Fig. 18 is a cross-sectional view of a conductive via in a substrate;
Figs. 19(a) and (b) are cross-sectional views of vias through a substrate prior to coating with a metal film;
Figs. 20(a) to (g) are schematic cross-sectional representations of method steps for forming a sensing device from a sheet having a metalised coating on one side, including forming and filling vias and, on the other side, forming a well array structure in the sheet;
Fig. 21 is a representation of a screenshot of current measurements taken during the blocking of a pore by an analyte (TBA), the pore having been inserted in a membrane supported on a sensing structure of the invention;
Figure 22(a) is a cross section view of a conductive via through a sheet having metal film on both sides, while Figure 22(b) is a cross section view of a conductive via through a sheet having metal film on one side and a screen-printed pad on the other;
Figure 23(a) is a schematic plan view of a track and pad on a surface of a substrate aligned with a sectional view of a conductive via connected to the pad, while Figure 23(b) is a schematic view of a cross-section and plan view of a pad having a capillary stop;
Figure 24 is a table and corresponding properties of sheet materials;
Figure 25 is a cross-section of layers forming a sensor upon a substrate formed using techniques disclosed herein;
Figures 26(a) to (d) are steps taken to fabricate an overhang in a single layer of material;
Figure 27 is a cross-section of a well formed in a substrate with an overhang layer in position to be attached thereto to create a well;
Figure 28 is a schematic top-down representation of a nanopore support structure having outer protrusions;
Figure 29(a) is a schematic top-down representation a nanopore support structure with outer protrusions, and figure 29(b) is a cross-sectional side view of a nanopore support structure with outer protrusions; and
Figure 30 is a top-down view of a nanopore support structure having outer protrusions having outer protrusions of an alternate form.

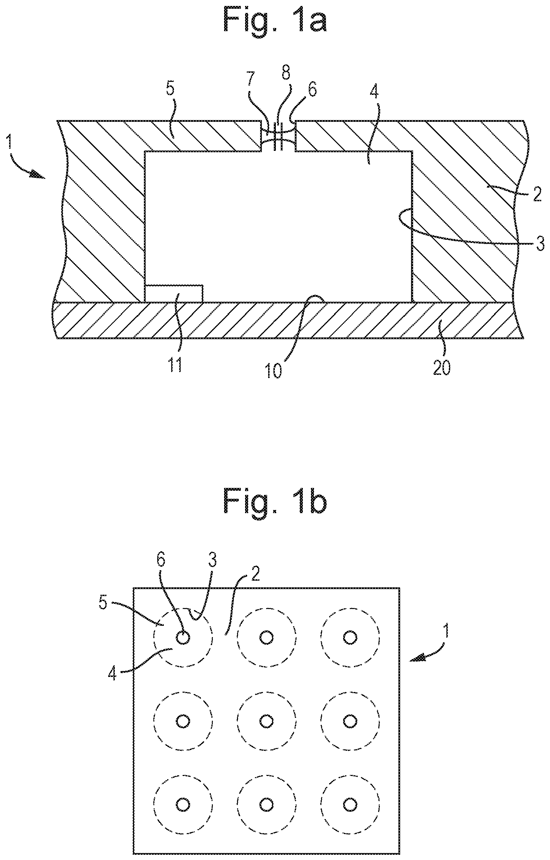

Figs. 1a and 1b show a nanopore support structure 1 in which known issues are reduced. The nanopore support structure 1 is designed to support a membrane 7 and thereby to support a nanopore 8 inserted in the membrane 7. The nanopore 8 is used to enable nanopore-based sensing, which may employ the measurement of ionic current flow through the nanopore 8. As molecules such as DNA translocate the nanopore 8, variations in the ionic current flow are produced that allow characterisation of the molecule. Examples of suitable membranes 7 and nanopores 8 are discussed further below.

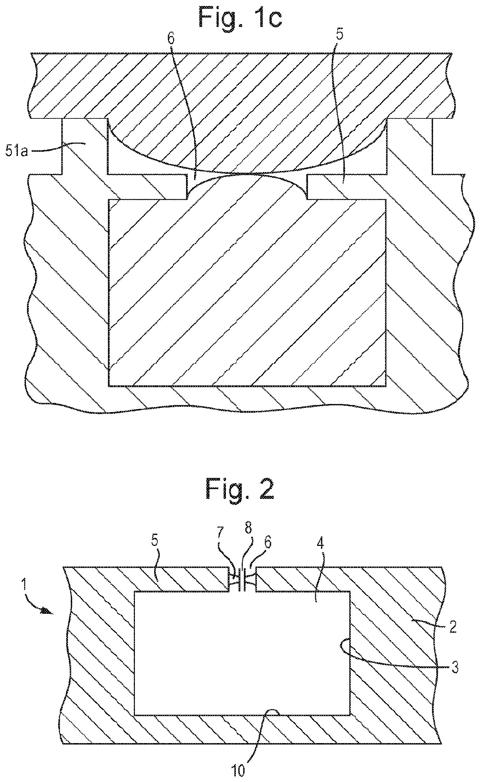

The nanopore support structure 1 comprises a wall layer 2 comprising walls 3 defining a plurality of wells 4. A single well is illustrated in Fig. 1a for clarity, but as shown in Fig. 1b plural wells 4 may be arranged in a plane in a regular planar array having a repeating structure when viewed along a longitudinal axis of the well 4 perpendicular to the plane of the array (vertical in Fig. 1). The array can be arranged in a rectilinear grid or hexagonal layout. Fig. 1b shows only nine wells 4 for simplicity, but in general the nanopore support structure 1 may have any number of wells, typically being much larger than nine, for example of the order of 1000 or more, and even up to 1000000 or more.

The walls 3 of the well 4 can extend perpendicularly from the base 11 of the well. The footprint of the wells 4 may be circular, but this is not essential and other shapes may be used, for example square, rectangular, elliptical or hexagonal. In some embodiments, the wells 4 have a substantially constant cross-section along at least one axis, for example having the shape of a prism. In some embodiments, the wells 4 may not have a constant cross-section. For example, the wells 4 may be in the form of hemi-spheres, or "dimples".

The nanopore support structure 1 comprises overhangs 5 extending from the walls 3 across each of the wells 4. The overhangs 5 define an upper extent of the wells 4 along the longitudinal axis of the wells 4. The overhang 5 extends in a plane parallel to the plane of the array of wells 4, and perpendicular to a longitudinal axis of the wells 4. In Fig. 1, where the wells 4 are cylindrical, the overhangs 5 extend perpendicular to the walls 3, although this may not be the case where the wells 4 have other shapes. The overhangs 5 extend inwardly from the walls 3 towards a centre of the well 4. The overhangs 5 may be substantially symmetric around the longitudinal axis of the well 4, so that they extend from the walls 3 by the same distance at all points around the well 4, but this is not essential and the wells 4 may have a different shape that is not symmetric around the longitudinal axis of the well 4.

The overhang 5 defines an aperture 6 configured to support a membrane 7 suitable for insertion of a nanopore 8. The aperture 6 may be circular, but may have other shapes in other embodiments, such as square. The shape of the aperture 6 can be independent of the shape of the well 4, such as the cross-section of the well.

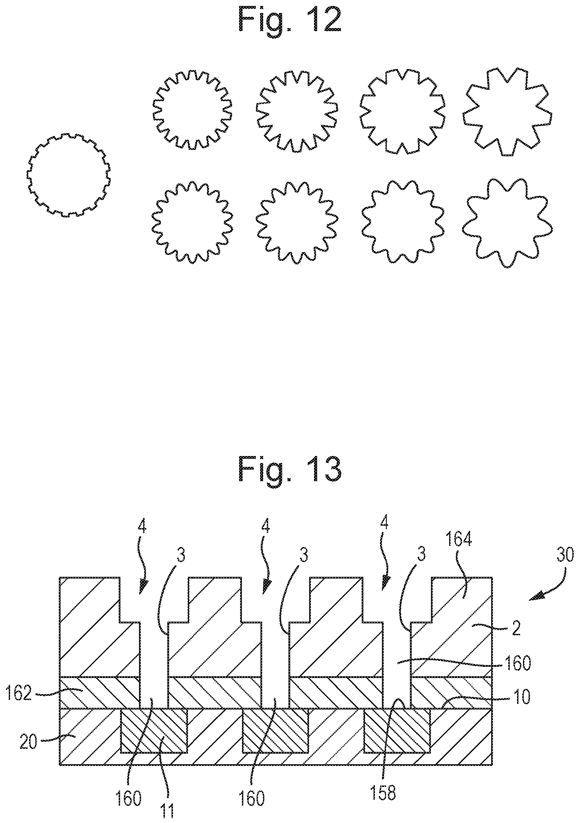

The aperture 6 may also have a more complex shape, for example having one or more portions around its perimeter that extend inwardly towards the centre of the aperture 6. Examples of such shapes are shown in Fig. 12, where the black portion indicates the aperture 6. These could take the form of a finger structure around the circumference of the aperture 6, or regularly spaced triangular features that give the aperture 6 the appearance of a cog wheel. Such structure would help to better deliver and control the oil which is used to promote membrane formation.

Using an aperture 6 defined by the overhangs 5 allows the size of the well 4 and the size of the membrane 7 needed to cover the well 4 to be decoupled. **In** particular, the size of the aperture 6 can be restricted relative to the diameter of the well 4. This restriction at the aperture 6 can reduce actual membrane size and area, so the capacitance of the membrane 7 is reduced, and the stability of the membrane 7 is improved.

It may also eliminate the need for a pre-treatment step when the membrane 7 is formed on the nanopore support structure 1. Such a pre-treatment step may comprise flowing an apolar medium over the nanopore support structure 1 to promote adhesion of the membrane 7 to the nanopore support structure 1. Having a restricted aperture 6 may make this step unnecessary.

The restricted aperture 6 can further balance the pressure across the membrane 7 and pins the membrane 7 to the location of the aperture 6. In particular, the top and bottom surfaces of the membrane 7 may extend from the same surface (i.e. the end surface of the overhang 5 that defines the aperture 6). This reduces the depth of the membrane 7 and improves the symmetry of the membrane 7 along the longitudinal axis of the well, which in turn reduces any bias that can lead to migration of the membrane 7 and draining of the well 4 in use, as described below. This allows the use of wells 4 with openings or through holes at the base 10 of the well 4.

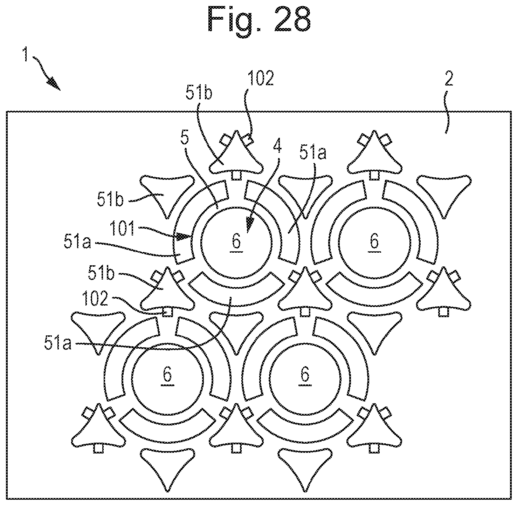

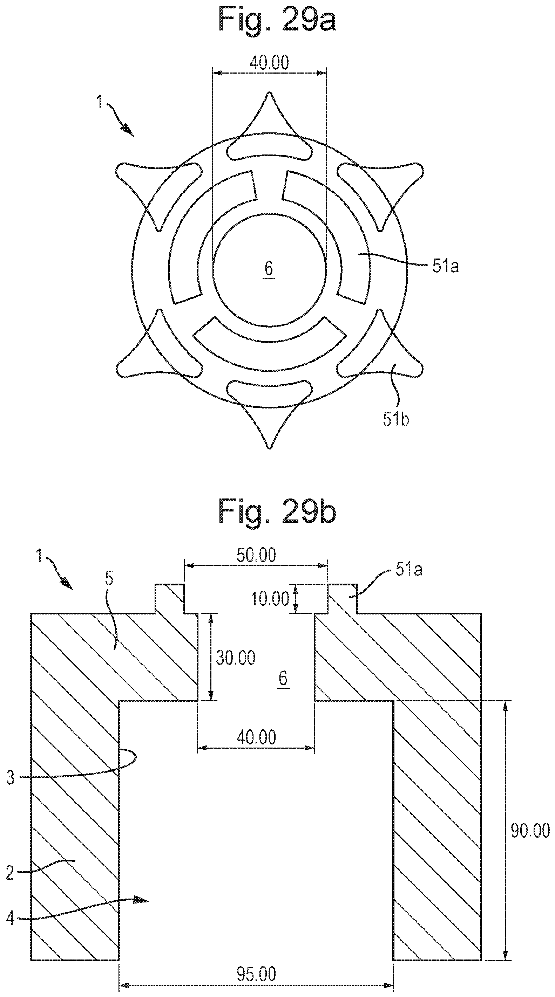

The bilayer in a well can be configured as shown in Fig. 1c, wherein the meniscus of the fluid in the well protrudes through, at least in part, the aperture 6 while the meniscus of the fluid in the cis chamber extends towards the aperture to form a bilayer interface. The bilayer interface forming the membrane can proximate the region of the aperture defined by the planes defined by the surfaces of the overhang 5. The bilayer interface, and can be level with the upper edge of the aperture. The menisci forming the membrane can be asymmetric. Fig. 1c shows outer protrusions 51a, discussed below in relation to Figs. 28 and 29.

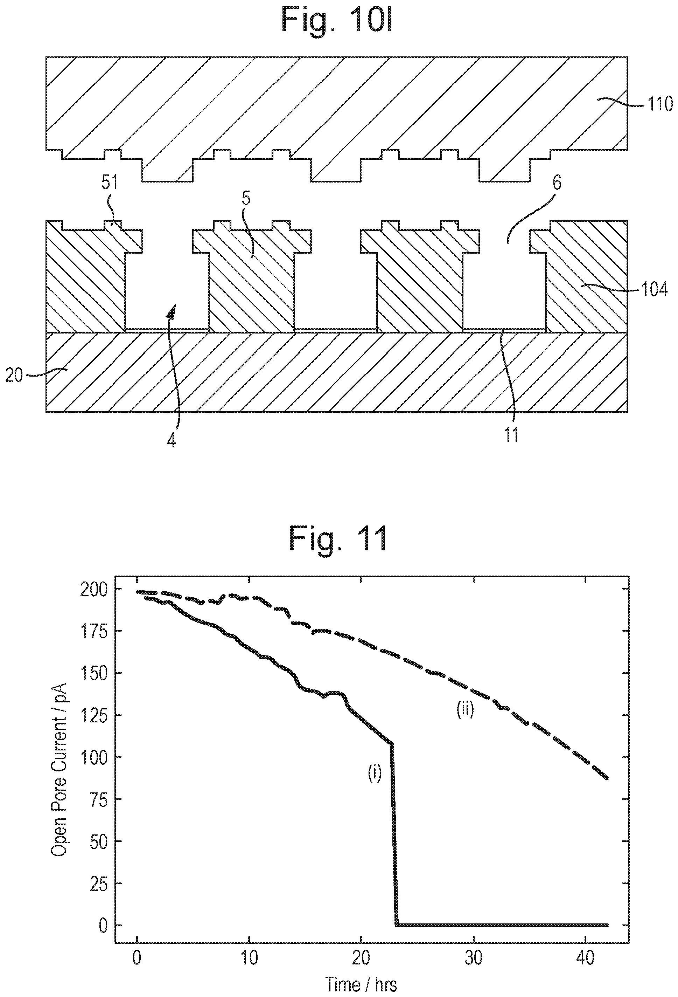

Further, the size of the well 4 can be increased without requiring a larger membrane 7, which is advantageous in many situations. In particular, this can allow a higher ionic current to be maintained through the nanopore 8 for a longer period of time. Fig. 11 compares (i) the open pore current measured through a conventional design of nanopore support structure 1, which lacks overhangs 5, as shown in the lowermost trace, and (ii) a nanopore support structure 1 with overhangs 5 defining apertures 6 according to the present invention, as shown in the uppermost trace. The conventional design suffers more rapid drop-off of open pore current, which falls abruptly to zero after less than 25 hours as the mediator solution is depleted. In contrast, the open pore current through the nanopore support structure 1 with overhangs 5 declines more slowly and is still at approximately 50% of its original value even after 40 hours. This problem of depletion of the solution can also be addressed by providing a second chamber 32 separate from the well 4, which will be discussed further below.

The size of the well 4 may in general be increased by increasing the depth and/or increasing the cross-sectional area (i.e. the footprint) transverse to the depth. However, increase of the cross-sectional reduces the density of apertures 6 across the array, so preferably the depth is increased in order to provide a high-density array.

Typically, the aspect ratio of width:depth of the well may be at least 1:3, preferably at least 1:5 and more preferably at least 1:10. The "width" in this context may be the diameter in the case of a well 4 that is circular, or may be a characteristic width such as the square root of the cross-sectional area.

Typically, the aspect ratio of the width of the aperture to the width of the well may be at least 2:3, preferably in the range of 1:2 and 1:5, although can be up to 1:10. The "width" in this context may be the diameter in the case of a well 4 that is circular, or may be a characteristic width such as the square root of the cross-sectional area.

Typically, the wells 4 can have a depth of at least 50µm, and preferably at least 120µm, but this is not limitative, and the wells can have a depth, for example, of up to 1mm.

The height of the pillars can range between 5µm and 30µm, such that the aspect ratio of the pillar height to the well depth can range between 1:10 and 1:200.

The wells 4 have respective bases 10. The bases 10 define the lower extent of the wells 4 along the longitudinal axis of the wells 4. The bases 10 extend in a plane parallel to the plane of the array of wells 4. In Fig. 1, the base 10 is perpendicular to the walls 3 of the nanopore support structure 1, although in general this may not be the case depending on the shape of the wells 4.

The nanopore support structure 1 further comprises a substrate 20. The substrate 20 may be formed from a different material to the wall layer 2. For example, the substrate 20 may be formed from silicon or plastic, for example Kapton^{™}. The wall layer 2 is fixed to the substrate 20 and the substrate 20 defines the bases 10 of the wells 4. The wall layer 2 may be formed directly on the substrate 20, or may be formed separately and fixed to the substrate 20 in a subsequent step.

The nanopore support structure 1 can comprise an electrode 11 formed on the base 10 of the well 4, and the well can be filled with an ionic solution, which can be an aqueous solution. The ionic solution can comprise a soluble electrode mediator e.g. ferri/ferrocyanide.

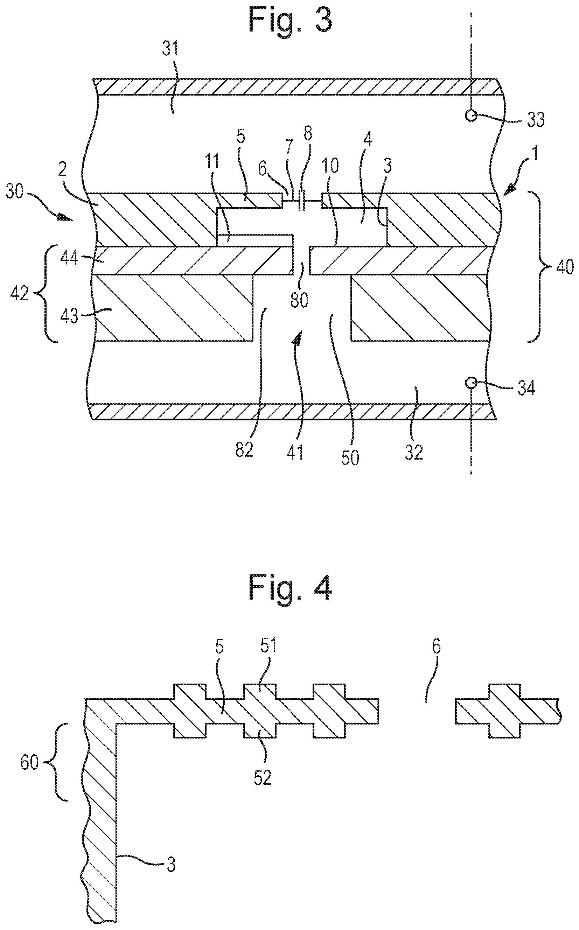

In an example where the well 4 is closed, the electrode 11 can be used to measure the ionic current in the well 4 relative to a reference outside the well 4. In an example where the well forms part of a passage, for example of the type shown in Fig. 3, the electrode 11 can be used to measure the fluidic potential in the well 4.

Fig. 1a shows a membrane 7 extending across a respective aperture 6 and a nanopore 8 inserted in the membrane 7. The nanopores 8 may be biological nanopores and the membranes 7 are capable of having the biological nanopores inserted therein. The membranes 7 may be amphiphilic membranes, for example formed from a phospholipid bilayer. However, a nanopore sensing device 30 comprising a nanopore support structure 1 according to the present invention may be provided without the membranes 7 and nanopores 8. In this case the end user carries out the steps to form the membranes 7 and cause the nanopores 8 to insert therein.

Fig. 2 shows a nanopore support structure 1 having an alternate form in which the wells 4 have respective bases 10, but the wall layer 2 further defines the bases 10. In this case the base 10 and the wall layer 2 are formed from the same material, and may be formed in the same manufacturing step, although this is not essential. Even where the wall layer 2 defines the base 10, the nanopore support structure 1 may still comprise a substrate 20, with the wall layer 2 fixed to the substrate 20.

A nanopore support structure 1 as shown in Fig. 1 or 2 may be incorporated into a nanopore sensing device. One possible approach when the well 4 is closed is to provide the nanopore support structure 1 with the aperture 6 opening into a chamber 25, for example as shown in Fig. 1.

Another approach is to arrange the nanopore support structure 1 between two chambers with the well 4 arranged to form part of a passage between those chambers, an example of which will now be described.

Fig. 3 shows a nanopore sensing device 30 that is arranged as follows. The nanopore sensing device 30 comprises a first chamber 31 and a second chamber 32 with a planar structure 40 between the first and second chambers 31, 32. The first and second chambers 31, 32 are filled with fluid. The first and second chambers 31, 32 are shown schematically in Fig. 3 but may be arranged with any suitable structure. The first and second chambers 31, 32 may be closed or may arranged as part of flow cells permitting flow of solution therethrough. In Fig. 3, the well 4 is separate from both the first chamber 31 and second chamber 32. However, in some embodiments, such as shown in Figs. 1 and 2, the second chamber 32 is provided by the well 4 itself. Where the well 4 is separate from the second chamber 32, each well 4 may have its own second chamber 32, or alternatively one or more wells 4 may share a common second chamber 32. In some embodiments, all of the wells 4 of the nanopore sensing device 30 may share a single second chamber 32.

The planar structure 40 is provided with plural fluidic passages 41 that extend between the first and second chambers 31, 32. Thus, the fluidic passages 41 are filled with fluid and fluidically connect the first and second chambers 31, 32. Each of the fluidic passages 41 is connected to the first and second chambers 31, 32, so the nanopore 8 lies in parallel paths of fluidic communication. The plural fluidic passages 41 may be arranged in an array in two dimensions across the planar structure 40.

In Fig. 3, the construction of the planar structure 40 is not shown, and so the fluidic passages 41 are shown schematically. The configuration of the planar structure 40 and the fluidic passages 41 is described in detail below.

In this example, as the first and second chambers 31, 32 are on opposite sides of the planar structure 40, the fluidic passages 41 extend through the planar structure 40. However, as an alternative, the first and second chambers 31, 32 could be arranged in different locations on the same side of the planar structure 40.

In this example, drive electrodes 33, 34 are provided in the first and second chambers 31, 32. In use, an electrical potential difference may be applied across the drive electrodes 33, 34 and therefore across each fluidic passage 41 to induce an analyte to flow between the first and second chambers 31, 32. The drive electrodes 33, 34 may be configured to apply substantially the same potential difference across all the fluidic passages 41. Additionally, or alternatively, the nanopore sensing device 30 can be configured to induce an analyte flow through the fluidic passages 41 using other techniques.

The first chamber 31 may function as a cis chamber and hold an analyte to be analysed by the nanopore sensing device 30. The second chamber 32 may function as a trans chamber and receive the analyte from the first chamber 31.

Thus, the planar structure 40 supports nanopores 8 in membranes 7 that extend across respective fluidic passages 41.

The fluidic passages 41 are each provided with an electrode 11 arranged to sense a fluidic electrical potential in the respective fluidic passage 41 between the nanopores 8 and the second chamber 32. As an analyte passes through a nanopore 8, the fluctuation in electrical potential caused by changes in ion current flow is detected by the electrode 11. Thus, the nanopore 8 and electrode 11 in a given fluidic passage 41 act as respective sensors in the nanopore sensor device 30. The planar structure 40 comprises a nanopore support structure 1 and a base layer 42 which are fixed together. As shown in Fig. 3, the base layer 42 replaces the substrate 20 of the nanopore support structure 1 in this example, but as an alternative, the entire nanopore support structure 1 as shown in Figs. 1 or 2 could be fixed to the base layer 42.

The nanopore support structure 1 is configured to support the nanopores 8 in the membranes 7 extending across the fluidic passages 41. In particular, the nanopore support structure 1 is provided with wells 4 opening into the first chamber 31. The wells 4 form part of the fluidic passages 41 and therefore have openings in their bases 10.

As already described, the wells 4 are configured to support the membranes 7 extending across the fluidic passages 41, specifically extending across the openings of the wells 4, and thereby to support the nanopores 8. The nanopore support structure 1 includes a wall layer 2 comprising walls 3 that define the wells 4.

The base layer 42 is between the nanopore support structure 1 and the second chamber 32 and includes the following layers. The base layer 42 includes a semiconductor wafer 43, which is formed by the substrate 20 of the nanopore support structure 1 in this example. The semiconductor wafer 43 supports a circuit layer 44. The circuit layer 44 comprises circuit components connected to the electrodes 11. The circuit components may allow the fluidic potential at the electrodes 11 to be sensed by an external circuit.

The fluidic passage 41 can provide the function of a fluidic resistor between the electrode 11 and the second chamber 32. Between is used in the sense of fluidic movement, such that the flow path between the electrode 11 and the second chamber 32 comprises the fluidic resistor portions 50. The fluidic resistor portions 50 may not be entirely physically located between the electrodes 11 and second chamber 32 in all embodiments. More specifically, the fluidic passage 41 extends through the base layer 42 and includes the well 4 as described above, fluidically connected in series with the fluidic resistor portion 50 formed in the base layer 42. The fluidic resistor portion comprises a circuit layer access hole 80 extending through the circuit layer 44 and a wafer access hole 82 extending through the semiconductor wafer 43 and fluidically connected to the circuit layer access hole 80.

The fluidic resistor portion 50 is configured to form a fluidic resistor. In this manner, the fluidic passages 41 are configured to provide fluidic resistors between the electrode 11 and the second chamber 32.

The wafer access hole 82 forms part of the fluidic resistor portion 50 and fluidic passage 41, and its dimensions can be configured to determine the fluidic resistance of the fluidic resistor portion 50. The wafer access hole 82 is structurally and geometrically configured to function as a fluidic resistor. This can be achieved by defining the aspect ratio of the wafer access hole 82. Additionally, or alternatively other techniques for implementing fluidic resistance in the wafer access hole 82 can be used, such as configuring the dimensions of the circuit layer access hole 80 to function as a fluidic resistor.

The fluidic resistance of the wafer access hole 82 can be varied by varying its dimensions, in particular its aspect ratio and by varying the ionic concentrations of the fluids in the first and second chambers 31, 32. For example, the wafer access hole 82 can be configured with a high aspect ratio to increase the resistance. Additionally, or alternatively, the fluid in the wafer access hole 82 can have a lower ionic concentration compared to the fluid in the first and second chambers 31, 32 to increase the resistance of the wafer access hole 82. Maintaining a higher ionic concentration in the first and second chambers 31, 32 improves the signal to noise ratio.

As a result, the fluidic passage 41 forms a voltage divider across the electrode 11.

The resistance of the nanopore 8 is present in a first leg of the voltage divider between the electrode 11 and the first chamber 31. The well 4 is designed to have a minimal fluidic resistance compared to the nanopore 8.

The fluidic resistance of the fluidic resistor portion 50 is present in the second leg of the voltage divider between the electrode 11 and the second chamber 32. The circuit layer access hole 80 and the wafer access hole 82 may be designed to provide a fluidic resistance that is negligible compared to the fluidic resistance of the fluidic resistor portion 50, but this is not essential and they may be designed to provide additional fluidic resistance.

As a result of the voltage divider, the fluidic electrical potentials sensed in the fluidic passage 41 by the electrode 11 permits sensing of the current flowing through the fluidic passage 41 and hence the nanopore 8. This allows for nanopore sensing.

The fluidic passage 41, and in particular the fluidic resistor portion 50, can be configured such that the resistance of the two legs of the voltage divider are substantially matched when the fluidic passage 41 is filled by fluid, and relatively high relative to the resistance of fluid in the first and second chambers 31, 32 such that the resistance of the first and second chambers 31, 32 does not appreciably influence the measurements.

The signal-to-noise ratio may be optimised by selecting the fluidic resistances of the two legs of the voltage divider to be equal. However, this is not essential and the fluidic resistance of the fluidic resistor portion 50 may be varied to take account of other factors, while still obtaining an acceptable signal-to-noise ratio. An acceptable signal-to-noise ratio may be achieved with the fluidic resistance of the second leg of the voltage divider being significantly less than the resistance of first leg of the voltage divider, for example with the fluidic resistance of the second leg of the voltage divider being 10% or less of the resistance of first leg of the voltage divider, for example 2% thereof. In some embodiments, a lower limit on the fluidic resistance of the second leg of the voltage divider may be set by the desired signal to noise ratio.

Other factors that may be considered in the selection of the fluidic resistance of the second leg of the voltage divider, and specifically the fluidic resistor portion 50, are as follows.

As the fluidic resistance of the second leg increases, the diffusion of ions decreases, causing increased depletion of ions near the pore, and thereby causing a decay of the signal over the timescale of a typical event over which a signal is obtained. In order to increase the read time available, which is limited by this effect, the fluidic resistance of the fluidic resistor portion 150 may be reduced. In many embodiments, this factor may place an upper limit on the fluidic resistance of the fluidic resistor portion 50.

As the fluidic resistance of the fluidic resistor portion 50 increases, the variation in the voltage across the nanopore 8 increases, which can complicate signal processing. In order to limit this effect, the fluidic resistance of the fluidic resistor portion 50 may be reduced. Reducing the fluidic resistance of the fluidic resistor portion 50 may increase bandwidth or provide leeway for additional capacitance in the fluidic passage 41.

Taking into account these factors, the fluidic resistance of the second leg of the voltage divider may be less than the resistance of the nanopore 8, typically at most 50%, or at most 25% of the resistance of the nanopore 8. In some embodiments, the optimal fluidic resistance of the second leg of the voltage divider may be around 10% of the resistance of the nanopore 8.

When reducing the ratio of the fluidic resistance of the second leg of the voltage divider to the resistance of the first leg of the voltage divider, the signal to noise ratio does not scale directly with that resistance ratio. For example, in some embodiments when the fluidic resistance of the second leg of the voltage divider is around 10% of the resistance of the nanopore 8, then the signal to noise ratio is around 30% of its optimal value.

The overhang illustrated in Fig.3 can be implemented upon sensing devices described and shown in WO2020/183172.

There will now be described various methods of manufacture of the nanopore support structure 1 described above. The methods specifically refer to the nanopore support structure 1 shown in Fig. 1 in which the bases 10 of the wells 4 are formed by a separate substrate 20, but could equally be adapted to the to the nanopore support structure 1 shown in Fig. 2 in which the bases 10 of the wells are formed by a portion of the wall layer 2.

As shown in the example of Fig. 4, some of the methods result in the overhangs 5 additionally comprising protrusions 51, 52 protruding laterally of the extent of the overhangs 5. As discussed further below, in some embodiments this may be achieved by depositing (or laminating) additional layers of photoresist material on top of the overhangs 5. The protrusions 51, 52 may act to support the membrane 7 or control where oil (or more generally apolar medium) is retained on the nanopore support structure 1. For this reason, the protrusions 51, 52 may also be referred to as oil control features (or more generally apolar medium control features). In some embodiments, the oil retention properties of the protrusions 51, 52 may help to prevent oil covering the electrodes 11, which may reduce their electrical performance.

The walls 3 of the wells 4 may also be designed having regard to control of oil (or more generally apolar medium).

In some embodiments, the walls 3 may be featureless. This can be because the walls would not be purposively exposed to apolar medium during the process of filling the wells, forming a membrane and inserting nanopores in the membrane - so no control feature is required. This can reduce the collection of apolar medium on the walls 3 which may be advantageous in some circumstances, for example to reduce the collection of apolar medium on the electrode 11.

In other embodiments, the walls 3 may be provided with apolar medium control features such as projections. Such apolar medium control features may extend across the entirety of the walls 3 from the overhang 5 to the base 11, or may be provided only in a part of the walls 3 adjacent the overhang 5, for example the region 60 in the example of Fig. 4. By way of example, protrusions 52 can be provided at the interface between the overhang 5 and the wall of the well 4. By way of example, the walls of the well 3 can incorporate fin-like features like those disclosed in US2015/265994.

The protrusions 51, 52 comprise inner protrusions 52 protruding laterally of the extent of the overhang 5 inside the respective wells 4, and outer protrusions 51 protruding laterally of the extent of the overhang 5 outside the respective wells 4. The protrusions 51, 52 extend in opposite directions along the same axis, which is the same as the longitudinal axis of the well 4. However, this is not essential, and in general the outer and inner protrusions 51, 52 may extend along different axes from one another and/or along an axis different to the longitudinal axis of the well 4. The protrusions 51, 52 in Fig. 4 have a substantially square shapes, but other shapes are also possible depending on the specific application or intended functionality of the protrusions 51, 52.

Typical protrusions 51, 52 are arrays of small pillars, or array of pillars with small fingers. In particular, the protrusions 51, 52 may be used to increase retention of apolar medium by the overhangs 5. This is advantageous in promoting correct formation of the membrane 7 in the aperture 6. Additionally, or alternatively, the protrusions 51, 52 may be used to increase rigidity of the overhangs 5. Rigidity may be increased by forming ridges on the overhangs 5 that are straight or curved, for example extending from the wall 3 onto the overhang 5 (in the case of inner protrusions 52) or extending from above the wall 3 onto the overhang 5 (in the case of outer protrusions 51).

Although the embodiment in Fig. 4 comprises both inner protrusions 52 and outer protrusions 51, as an alternative, the overhang 5 may comprise only inner protrusions 52 or only outer protrusions 51.

In some embodiments, the outer protrusions 51 may be formed to define a protrusion wall 101 at least partially surrounding each aperture 6. Figure 28 shows such an embodiment. In such embodiments, the outer protrusions 51 may protrude laterally of the extent of the overhang 5 outside the respective wells 4 and/or laterally of the extent of an outer surface of the wall layer 2 between the wells 4 (i.e. the surface of the wall layer 31 facing the first chamber 31). The outer protrusions 51, formed on either the wall layer 2 or the overhang 5, may be formed using the processes described in more detail below.

Figure 28 shows a top-down view of a nanopore support structure 1, comprising a number of wells 4, with respective overhangs 4 defining apertures 6. For clarity, only one of the illustrated wells 4 is fully labelled in the drawing. Although only four wells 4 are illustrated, it is to be appreciated that any number of wells 4 and corresponding features may be used.

For each aperture 6, the outer protrusions 51 comprise a plurality of peripheral outer protrusions 51 defining a respective protrusion wall 101 at least partially surrounding and set back from the aperture 6. In the illustrated embodiment the outer protrusions 51 comprise, for each aperture 6, a plurality of peripheral outer protrusions 51a. The peripheral outer protrusions 51a are arranged partially around their respective aperture 6, leaving gaps between the peripheral outer protrusions 51a. The peripheral outer protrusions 51a comprise an inner surface facing towards the aperture that defines the protrusion wall 101, and an outer surface facing away from the aperture. The peripheral outer protrusions 51a can be arranged concentrically around the aperture.

In the illustrated embodiment, three peripheral outer protrusions 51a are provided for each aperture 6. Any other number of peripheral outer protrusions 51a may be used to define the peripheral wall 101 around each aperture, including one. The number of peripheral outer protrusions 51a may vary between 2 and 20. One or more of the peripheral outer protrusions 51a may be common to multiple apertures 6.

The protrusion wall 101 is configured to enable a meniscus to be formed across the respective aperture 6 such that the meniscus extends, at least in part, into the respective aperture 6. In particular, the height of the protrusion wall 101 above the aperture, and/or the distance between the aperture 6 and the protrusion wall (i.e. how set back the protrusion wall 101 is from the edge of the aperture 6) may be configured to enable a meniscus to be formed extending at least in part into the aperture 6. In general, the protrusion wall 6 may be such that an interface between an upper fluid and a lower fluid is in theFakir state. The conditions for forming a Fakir state are discussed in Afferrante et al., J. Phys.: Condens. Matter 22 (2010) 325107. This control of the meniscus may be used to assist formation of membranes in the aperture, and may avoid the need for pre-treatment to promote membrane formation.

In the illustrated embodiment, the peripheral outer protrusions 51a are arranged with gaps therebetween, so that the protrusion wall 101 only partially surrounds the aperture. While the protrusion walls 101 are shown in Fig. 28 surrounding approximately 85% of the aperture, the gaps within the walls can vary in size such that the protrusion walls surround between 75% to 95% inclusive of the aperture 6. Here, the percentage surrounded equates to the ratio of the total azimuthal angular extent of the protrusion wall 101 (when considered in polar coordinates with respect to the centre of the aperture 6), relative to a full circle (i.e. 360°). Such coverage extents provide a balance between controlling the meniscus within the peripheral wall 101, and allowing flow of the apolar medium towards the aperture 6 through the gaps.

The outer protrusions 51 of the illustrated embodiment further comprise intermediate outer protrusions 51b. The intermediate outer protrusions 51b are arranged in an area between the peripheral outer protrusions 51a. The intermediate outer protrusions 51b further assist flow of the apolar medium and limiting buffer liquid wetting to within the protrusion walls 101. The illustrated intermediate protrusions 51b have a substantially triangular cross-section, but other shapes of cross-section may be used.

Figs. 29a and 29b illustrate further views of the nanopore support structure 1 of Fig. 28. Fig. 29a shows the outer protrusions 51a, 51b around a single aperture 6. As can be seen, both the peripheral outer protrusions 51a and the intermediate outer protrusions 51b are arranged concentrically around the aperture 6. Although the intermediate outer protrusions 51b may be shared between multiple apertures 6, as in Fig. 28, when viewed from the perspective of a single aperture 6 a set of intermediate outer protrusions 51b may be arranged around the aperture 6, concentrically with the aperture 6 and peripheral outer walls 51a.

Fig. 29b illustrates a cross-section through an individual well 4, showing peripheral outer protrusions 51b for that well 4. Figs. 29a and 29b illustrate example relative dimensions of the various components of a nanopore support structure 1. It is to be appreciated that these dimensions are provided only as an example, and are not intended to be limiting.

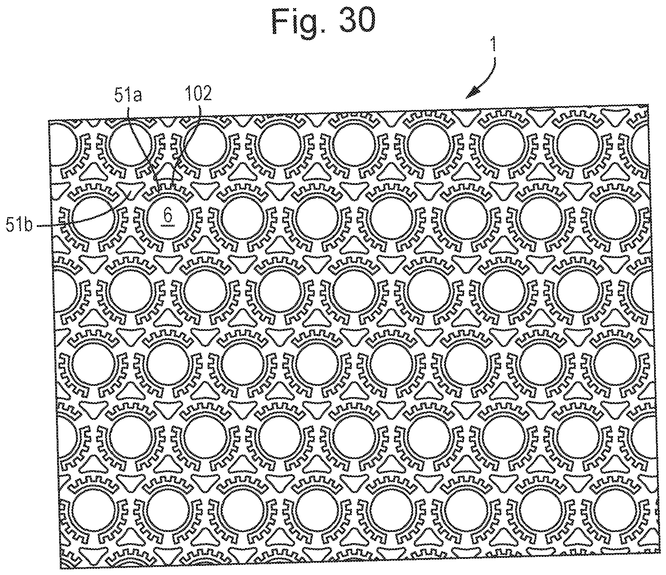

In the embodiment of figure 28, the inner and outer surfaces of the peripheral outer protrusions 51a are substantially smooth. In other embodiments, the inner and/or outer surfaces may comprise micropatterned structures. Figure 30 shows such an embodiment, wherein the outer surfaces of peripheral outer protrusions 51a have micropatterned structures 102. The micropatterned structures 102 may further assist flow of the apolar medium, and limit wetting away from the apertures 6. The micropatterning may be formed by treatment of moulded structures, where moulded structures are used to form the outer protrusions 51, as described in more detail below. Micropatterning may also be used on one or more surfaces of one or more intermediate outer protrusions 51a. By way of example, micropatterned structures are provided on the intermediate outer protrusions 51b of Figure 28 as teeth having a substantially square profile, although other shapes can be implemented. The micropatterned structures 102 can retain oil used in forming the membrane. The gaps between the peripheral outer protrusions 51a can facilitate the flow of oil used in forming the membrane across the surface of the well array. To inhibit the membrane descending in the gaps and the membrane rupturing, the micropatterned structures 102 can be configured aligned with the gaps, and function to support the membrane, while enabling oil to flow across the surface between the structures. Thus in some embodiments one or more intermediate outer protrusions 51b may comprise micropatterned structures 102 on one or more surfaces of the intermediate outer protrusions 51b, the micropatterned structures extending towards gaps between the peripheral outer protrusions 51a.

One type of method involves using multiple exposure steps with control of the exposure time, or use of a grayscale exposure technique, to form the overhang structure with only a single final develop step. First there will be described examples in which the nanopore support structure 1 is formed by methods using exposure and curing of negative photoresist material, so that the overhangs 5 and the wall layer 2 comprise cured, negative photoresist material. Such methods in general comprise depositing uncured negative photoresist material, exposing the negative photoresist material so as to cure the negative photoresist material in the form of the nanopore support structure 1, and removing the uncured negative photoresist material.

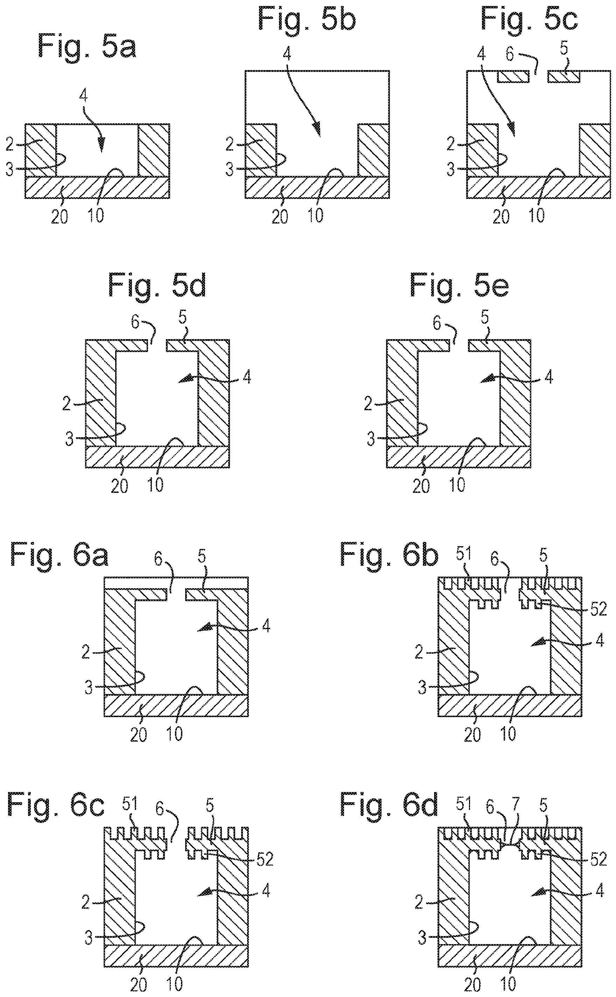

In some embodiments, the method comprises depositing uncured negative photoresist material, exposing the negative photoresist material with a multi-exposure technique so as to cure the negative photoresist material in the form of the nanopore support structure 1, and removing the uncured negative photoresist material. An example of this type of multi-exposure method is shown in Fig. 5. In this example, the nanopore support structure 1 is formed through multi-exposure of the overhang structure with only one final develop step.

In Fig. 5, the method comprises, prior to performing said steps of depositing uncured negative photoresist material and exposing the negative photoresist material with a multi-exposure technique, performing an initial stage comprising depositing an initial layer of uncured negative photoresist material, and exposing the initial layer of negative photoresist material so as to cure the negative photoresist material in the form of a lower section of the wall layer 2. This is shown in Fig. 5a, where the initial layer is exposed.

In Fig. 5b, the uncured negative photoresist material that is exposed with a multi-exposure technique is deposited as a further layer on the initial layer of uncured negative photoresist material. Subsequently, the step of exposing the negative photoresist material with a multi-exposure technique is carried out so as to cure the negative photoresist material in the form of the overhangs 5 and at least an upper section of the wall layer 2 to a deeper level than the overhangs 5.

In Fig. 5, the step of exposing the negative photoresist material with a multi-exposure technique comprises exposing the negative photoresist material in separate exposure steps. This is shown in Fig. 5c and Fig. 5d. In Fig. 5c, the negative photoresist material is cured in the form of the overhangs 5. In Fig. 5d, in a separate exposure step, the negative photoresist material is cured in the form of at least an upper section of the wall layer 2 to a deeper level than the overhangs 5. In Fig. 5e, a single develop step is performed for the whole stack of layers of negative photoresist material to remove all of the uncured photoresist material. The curing of the photoresist material to a deeper level in Fig. 5d than in Fig. 5c may be achieved in various ways. For example, the photoresist material could be exposed for a longer time in Fig. 5d than in Fig. 5c, or the intensity of the light used to expose the photoresist material may be higher in Fig. 5d than Fig. 5c.

This type of technique can be facilitated by the particular choice of photoresist material. For example, an advantageous property of thick, SU-8 like permanent photoresist material is that the exposure light penetrates from top to bottom gradually during the exposure process due to a change in the absorption coefficient of the material during/after exposure. This facilitates only exposing the top part of the resist layer with the correct choice of exposure timing. Combined with multiple exposures, this enables the overhangs 5 to be exposed while leaving unexposed photoresist material underneath. This means it is unnecessary to develop the initial layer before depositing the uncured negative photoresist material that is exposed with a multi-exposure technique, and the uncured photoresist material can naturally act as a support for photoresist material above during subsequent steps.

The multi-exposure technique is not limited to two different exposure levels provided in two separate exposure steps. An alternative approach is grayscale lithography/exposure. In this technique, the step of exposing the negative photoresist material with a multi-exposure technique comprises exposing the negative photoresist material with a spatial modulation in intensity. This can be used to form various thickness (up to the total resist thickness) of cured portion of the photoresist material depending on the exposure dosage. In this way, the cross-section of the aperture 6 can be tuned easily to promote correct membrane 7 formation.

As mentioned above, in some embodiments, the method comprises exposing the negative photoresist material so as to cure the negative photoresist material in the form of the overhangs 5 and protrusions 51, 52 protruding laterally of the extent of the overhangs 5. The protrusions may be inner protrusions 52 and/or outer protrusions 51.

In some embodiments where inner protrusions are formed, the method comprises exposing the negative photoresist material with a multi-exposure technique so as to cure the negative photoresist material in the form of the overhangs 5 and inner protrusions 52 protruding laterally of the extent of the overhangs 5 inside the respective wells 4. The inner protrusions 52 may be formed at the same time as the overhangs 5 in the multi-exposure technique. For example, in the step shown in Fig. 5c, a greater exposure intensity or exposure time may be provided at some points along the overhang 5 in order to expose the photoresist material to a greater depth and form the inner protrusion 52. Alternatively, the exposure intensity may be spatially modulated in a single exposure step. The multi-exposure or grayscale exposure techniques use the support of the unexposed photoresist material under the overhang 5 to allow formation of these protrusions not only on the top of the overhang surface, but also underneath the overhang 5. This can bring better membrane forming, better oil control and overall membrane stability.

Fig. 6 shows an embodiment of the method where both outer protrusions 51 and inner protrusions 52 are formed. In this embodiment, the method comprises depositing an overhang layer of uncured negative photoresist material and exposing the first layer of negative photoresist material so as to cure the negative photoresist material in the form of the overhangs 5. This corresponds to the steps shown in Figs. 5b to 5d, although it is not essential that the top portion of the walls 3 be formed at this time as well as shown in Fig. 5d. The top portion of the wall 3 may be formed after completely forming the overhangs 5 and protrusions 51, 52.

Following the formation of the overhangs, the method comprises depositing a top layer of uncured negative photoresist material on the overhang layer of uncured negative photoresist material, as shown in Fig. 6a. Then, a step of exposing the second layer of negative photoresist material so as to cure the negative photoresist material in the form of outer protrusions 51 protruding laterally of the extent outside the respective wells 4 is performed, as shown in Fig. 6b. In Fig. 6b, the inner protrusions 52 are formed at the same time as the outer protrusions 51. This can be achieved by exposing the photoresist material at a higher intensity or for a longer time in the regions where the inner protrusions 52 are to be formed.

In some embodiments, as shown in Fig. 6b, the outer protrusions 51 are directly above the inner protrusions 52. This is because the top layer of photoresist material will be exposed as well if inner protrusions 52 are to be formed. If the outer protrusions 51 are not directly above the inner protrusions 52, then the inner protrusions must be formed before the top layer of uncured negative photoresist material is deposited. The step of removing the uncured negative photoresist material is performed only after the negative photoresist has been cured in the form of the overhangs 5 and the protrusions 51, 52. This final development step is shown in Fig. 6c, and removes all unexposed (and therefore uncured) photoresist material. Following this, the membrane 7 can be formed across the aperture 6 using oil and polymer as shown in Fig. 6d. However, as mentioned above, the formation of the membrane may be performed by the end user, and so the step shown in Fig. 6d may not be performed prior to shipment of the product.

Outer protrusions 51 can be formed in-line with, off-set from or a combination thereof, from the inner protrusions 52. This can be achieved by a separate laminate and exposure step. When the nanopore support structure 1 comprises electrodes 11 to permit voltage sensing, each well 4 has an access hole at the bottom in the form of the fluidic passage 41 to connect to a fluidic resistor portion 50. If a membrane were to be formed across a well having an access hole using known structures, such as disclosed in US2015/265994, then additional pressure from surface tension of the membrane, or even pressure perturbations in the fluid can push liquid through the fluidic passage 41 and slowly drain the buffer solution from the well 4 until the membrane 7 collapses to the bottom of the well 4.

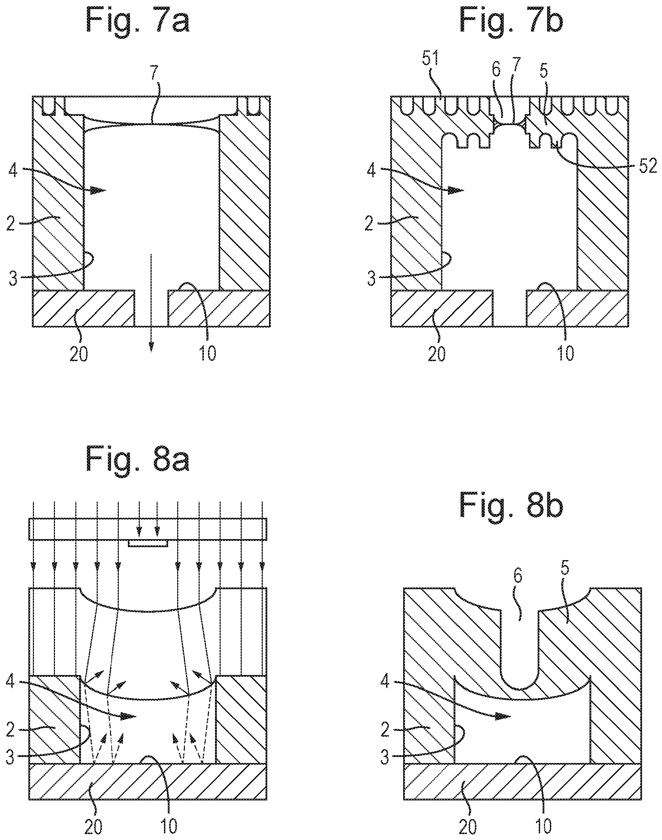

In contrast, with the overhang 5, the membrane 7 formed across the aperture 6 has at least one of (i) a reduced size, thus increasing its stability, (ii) minimal surface tension in its steady state condition, (iii) a mostly planar surface, and (iv) shallow depth, controllable by the thickness of the overhangs. Any small perturbation that pushes the membrane 7 away from the aperture 6 will increase the membrane size, which is energetically unfavourable. One or more of these features function to effectively pin the membrane 7 down at the location of the aperture 6 and can help forming long term stable membrane 7 for voltage sensing applications, as illustrated in Fig. 7.

Fig. 7a shows a nanopore support structure without overhangs 5. The large size of the membrane 7 means that the membrane 7 is not stable in the long term. In contrast, Fig. 7b shows a nanopore support structure 1 according to the present invention. The smaller aperture 6 means the membrane 7 is much smaller and more stable in the long term. In all examples discussed herein, the overhang 5 and aperture 6 structure removes the need for a surface coating of oil/membrane to penetrate to the bottom of the well 4 during use, as was necessary in previous nanopore support structures. As long as the oil/membrane mixture covers the overhang 5 (and any protrusions that are present, for example in the form of pillars/fingers as seen in Fig. 7b), the nanopore support structure 1 will provide the necessary oil control and membrane support function. This provides greater flexibility of choice of material in the well structure, because the well is not limited to hydrophobic materials to construct the wall layer 2 that were previously needed to promote oil adhesion. A hydrophilic wall layer 2 and/or base 10 may be helpful in terms of fluidic resistor filling and bubble tolerance. This can also eliminate pre-treatment steps in which oil is applied, and thereby simplify the process of assembling a nanopore sensing device 30 using the nanopore support structure 1. It is only necessary to vacuum or pressure fill the wells 4, drain and clip off the buffer solution from the front, add oil/membrane mixture to the top of the wells 4 where the overhang 5 and possible protrusions 51, 52 are present, and flow solution through the cis chamber.

Attempts to produce overhangs 5 using additional layers of photoresist material deposited after the initial layer is exposed and developed can create challenges that make the exposure process less reliable than it needs to be. Firstly, a lack of support under the additional layers of photoresist material suspended over the well 4 causes the additional layer to sag down across the well 4, making it bowed. Secondly, an optical cavity is formed due to the developed structure of the well 4. This causes light from subsequent exposure steps to be scattered off the boundaries of the cured photoresist material. As a result, a partially exposed thin film of cured photoresist material remains across the intended aperture 6 after the final development step, because of the refracted/scattered light into the not exposed region. These effects are illustrated in Fig. 8. Fig. 8a shows the subsequent layer of unexposed photoresist material sagging into the well 4. In addition to undesirable sagging, light is refracted/reflected toward the under-mask parts of the layer of photoresist material, causing exposure of undesired regions of the photoresist material. Consequently, following a subsequent development step, a partially exposed film of cured photoresist material seals the aperture 6 as shown in Fig. 8b.

One way to minimise these effects is the multi-exposure techniques mentioned already above. Alternatively, or additionally, additional support structures inside the well 4 can be provided in the first layer of photoresist material, so the sagging of subsequent layers of photoresist material can be reduced. These support structures may also be referred to as barriers 70. In such embodiments, the nanopore support structure 1 further comprises barriers 70 disposed in the wells 4, the barriers 70 being capable of reducing scattering of electromagnetic radiation of a wavelength for curing the negative photoresist material.

The method of forming a nanopore support structure 1 including barriers 70 comprises depositing a first layer of uncured negative photoresist material, exposing the first layer of negative photoresist material so as to cure the negative photoresist material in the form of the wall layer 2 and barriers 70 disposed in the wells 4, removing the uncured negative photoresist material of the first layer to form the wells 4 and the barriers 70, depositing a second layer of uncured negative photoresist material on the first layer of uncured negative photoresist material, exposing the second layer of negative photoresist material so as to cure the negative photoresist material in the form of the overhangs 5, the barriers 70 being shaped so as to reduce scatting of electromagnetic radiation applied in the exposure, and removing the uncured negative photoresist material of the second layer.

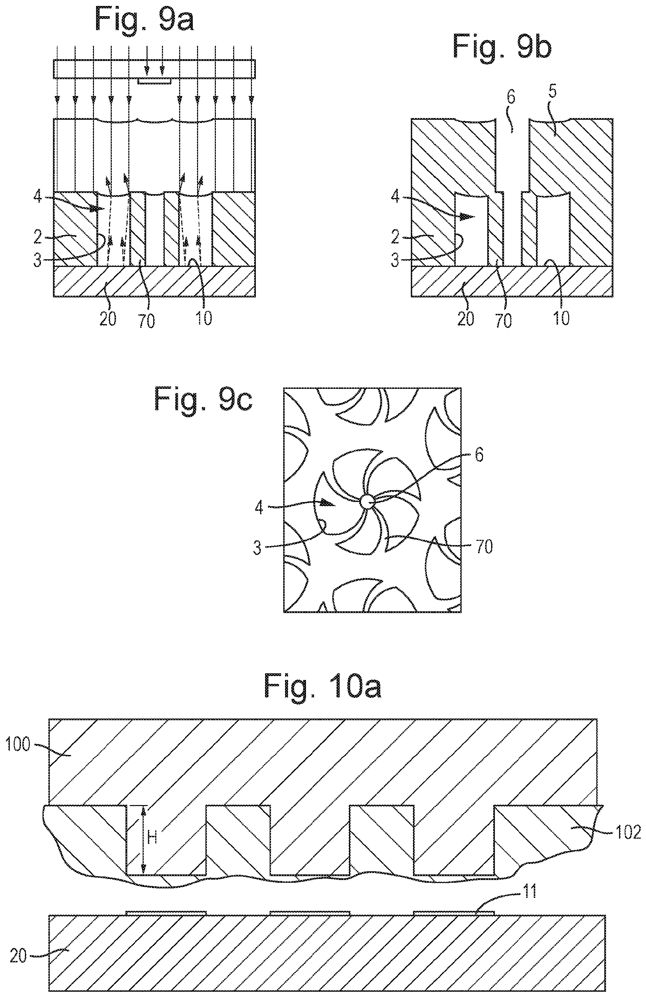

The barriers 70 reduce refraction/reflection of light in the well 4 and can also reduce the sagging down of the suspended photoresist material layer, as shown in Fig. 9a. Fig. 9b shows that, after development and removal of the uncured photoresist material, that aperture 6 is clear. In Fig. 9a and Fig. 9b, the wells 4 have respective bases 10 and the barriers 70 extend from the bases 10 to the overhangs 5.

For wells 4 which do not have a fluidic passage 41 at the bottom, the volume of the well 4 is very important, being proportional to the run time of the nanopore support structure 1. Therefore, it is desirable to minimize the barrier 70 volume without significantly affecting its rigidity. There are many ways to design a small volume rigid barrier 70 to hold the suspended layers of photoresist material. The barriers 70 mean that the suspended additional layers of photoresist material only cross much smaller distances and therefore do not sag. Fig. 9c shows an example of such barriers 70 in a top-down view of the nanopore support structure 1. The darker region shows the first layer of photoresist material, in which five-lobe curved barriers 70 are clearly visible. The barriers 70 extend from the walls 3 inwardly into the wells 4. The barrier 70 is designed to minimize the volume it takes and in Fig. 9c the barriers 70 are curved along their extent inwardly into the wells 4. The curvature increases the rigidity and help reflect/block light away from the centre of the well 4. The second layer of photoresist material covers both the dark and light green region, leaving a much smaller aperture 6 for forming the membrane 7.

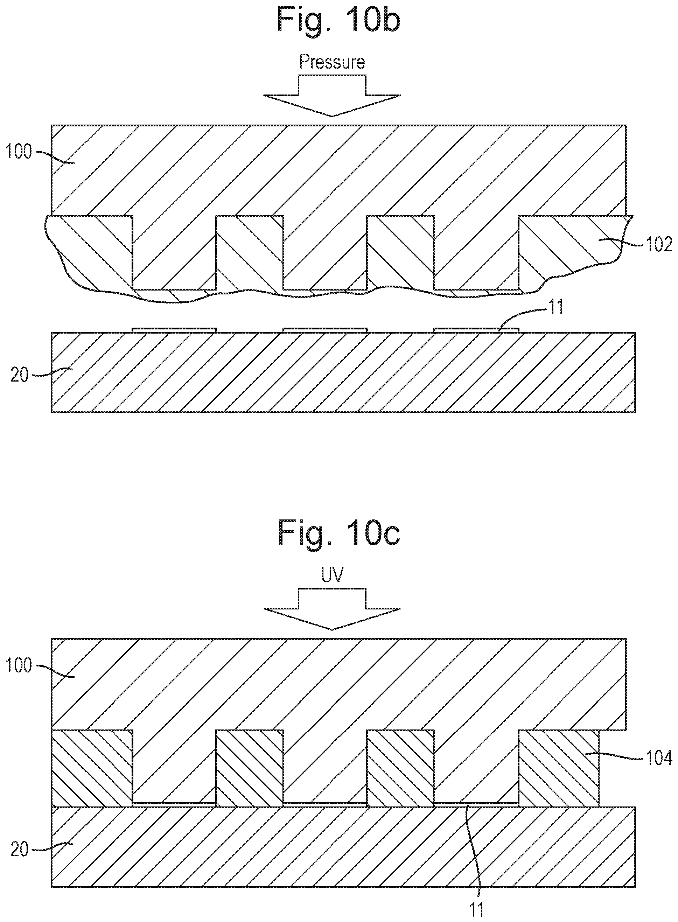

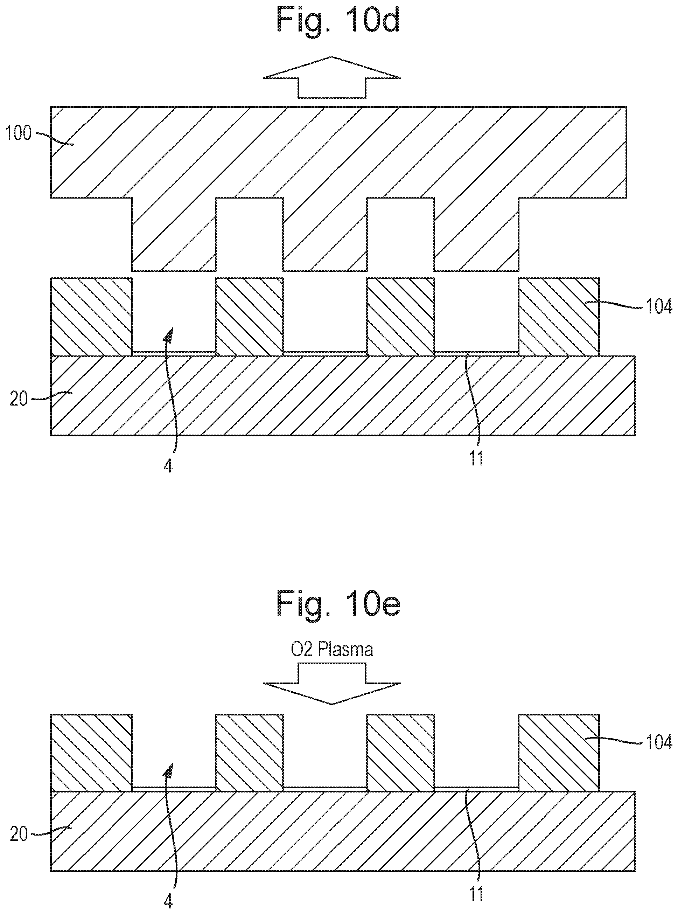

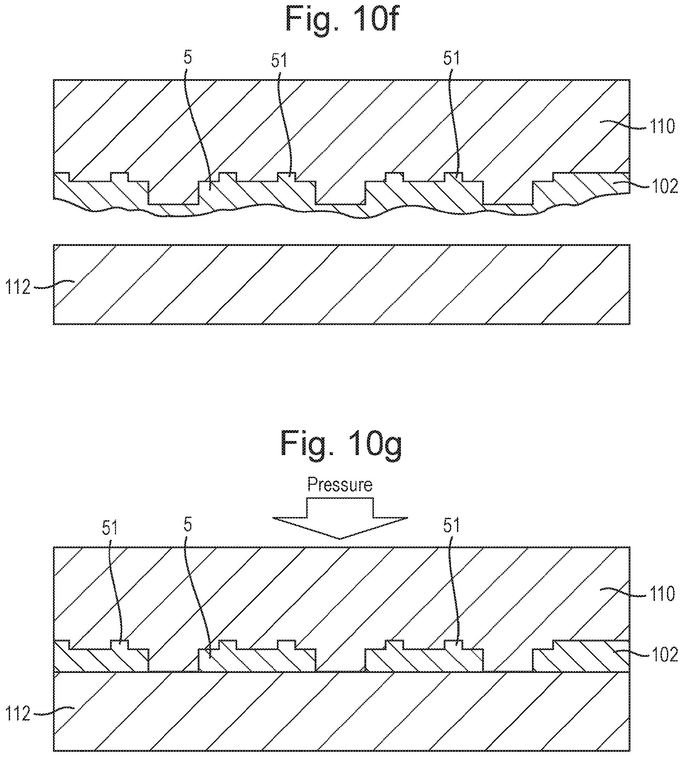

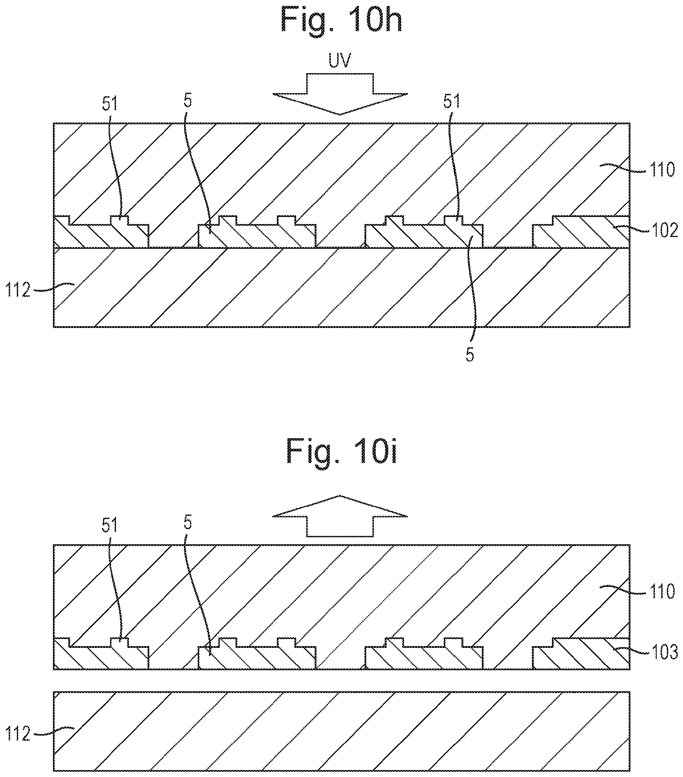

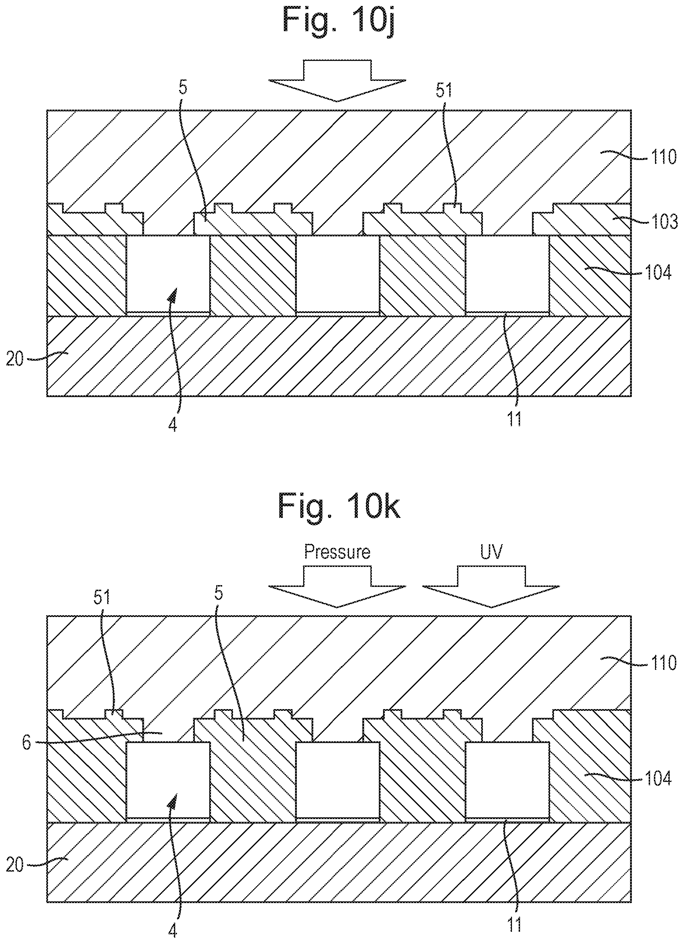

So far, all methods of forming the nanopore support structure 1 have involved exposure of layers of photoresist material. Alternatively, not according to the claimed invention, the nanopore support structure 1 may be formed by moulding. In this case, the wall layer 2 and the overhangs 5 are respective moulded components that are fixed together. An example of a method of manufacturing a nanopore support structure 1 using moulding is shown in Fig. 10. The method comprises forming the wall layer 2 comprising walls 3 defining a plurality of wells 4 and forming the overhangs 5 in separate steps, the overhangs 5 being fixed to the wall layer 2 so as to extend from the walls 3 across the wells 4.

The step of forming the wall layer 2 comprises moulding the wall layer 2 on a substrate 20, which in this example is formed of silicon. This is shown in Figs. 10a to 10d. In Fig. 10a, an uncured mouldant 102 is vacuum filled into a wall layer mould 100 formed from polydimethylsiloxane (PDMS). In this example, the height H of the walls 3 is approximately 90µm. The substrate 20 has electrodes 11 formed thereon, in this example formed of platinum. In Fig. 10b, the wall layer mould 100 is aligned with the electrodes 11 such that the wall layer 2 is moulded on the substrate 20 to locate the electrodes 11 in the wells 4. Pressure is applied to compress the uncured mouldant 102 onto the substrate 20 and into the wall layer mould.

In Fig. 10c, the mouldant is cured by irradiation with ultraviolet (UV), resulting in cured mouldant 104 in the shape of the wall layer 2. In this example, UV light with a wavelength of approximately 365nm is used. Depending on the choice of mouldant, other methods may be used to cure the mouldant 102, for example by heating the mouldant. In Fig. 10d, the wall layer mould 100 is removed. In some cases, this may leave a thin layer of cured mouldant 104 over the surface of the electrodes 11, as shown in Fig. 10d. This is undesirable, as it may impede the electrical performance of a nanopore sensing device 30 into which the nanopore support structure 1 is integrated. To remove this layer, a process known "descumming" is performed. This is illustrated in Fig. 10e, where an oxygen plasma or laser can be applied to clean the surface of the electrodes 11. This step may not be necessary in all embodiments of the method, depending on whether a layer of cured mouldant 104 is left on the electrodes 11.

Having formed the wall layer 2 on the substrate 20, the overhangs 5 are separately formed. This is illustrated in Figs. 10f to 10i. In Fig. 10f, uncured mouldant 102 is vacuum filled into an overhang mould 110, which is formed of PDMS. In this example, the overhangs have a thickness of approximately 30µm, and are configured such that the aperture 6 of the well 4 will be between about 10µm to about 60µm, and preferably about 40µm, once the overhangs are fixed to the wall layer 2. The overhang mould 110 is configured such that a plurality of overhangs 5 are formed simultaneously, with the correct alignment and spacing to be directly placed onto the corresponding walls 3 to define the apertures 6 of a plurality of well 4. This means that a plurality of well 4 can be formed simultaneously, thereby increasing manufacturing speed compared to forming overhangs 5 separately and having to align them individually with corresponding walls 3.

In Fig. 10g, the overhang mould 110 is compressed onto a base 112, which in this example is formed of the same PDMS material as the overhang mould 110. Pressure is applied to compress the uncured mouldant 102 into the overhang mould 110. Optionally, a partial curing step is performed as shown in Fig. 10h. This promotes retention of the correct shape of the overhangs 5, while still allowing some deformation of the overhangs 5 that can be used to fix them to the wall layer 2. In Fig. 10i, the overhang mould 110 is removed from the base 112, and the overhangs 5 stay with the overhang mould 110, as illustrated by the overhang portion 103. This is achieved by peeling the base 112 leaving the pre-aligned overhang mould 110 held on with the partially cured moulded array of overhangs 5 still present and ready to be aligned and fixed to the wall layer 2.

Figs. 10j to 10l illustrate how the overhangs 5 are fixed to the wall layer 2 in the step of forming the overhangs 5. Forming the overhangs 5 comprises moulding the overhangs 5 on the wall layer 2. In Fig. 10j, the overhang mould 110 with the overhangs 5 is aligned with the wall layer 2 on the substrate 20. Pressure is applied to compress the overhangs 5 against the top of the wall layer 2 once the overhang mould 110 touches down. In Fig. 10k, UV light is applied to cure the overhangs 5 and join them to the wall layer 2. In Fig. 10l, the overhangs mould 110 is removed, leaving the completed nanopore support structure 1. Optionally, an additional heating step may be performed to further cure the mouldant, although this can be reduced or omitted entirely. Additionally, or alternatively, an oxygen plasma cleaning step can be applied between steps 10i and 10j. Not only does this function to clean the surface of the electrodes 11, but the process leaves the surface of the cured mouldant 104 with adhesive properties that enhance the bond to the overhang portion 103.

In Figs. 10f to 10i, the step of forming the overhangs 5 comprises moulding the overhangs 5 with protrusions protruding laterally of the extent of the overhangs 5. In particular, the protrusions comprise outer protrusions 51 protruding away from the respective wells 4, and the step of forming the overhangs 5 comprises moulding the overhangs 5 with the outer protrusions on the wall layer 2. In this example, the outer protrusions 51 have a height above the overhang 5 of approximately 15µm. The protrusions may also comprise inner protrusions protruding laterally of the extent of the overhang 5 inside the respective wells 4.

Additionally or alternatively to the plasma cleaning of any residue on the surface of a moulded component e.g. the PDMS stamp may leave residual oligomeric DMS material on the surface of the mouldant leading to variability in the surface energies of moulded chips, solvent extraction can be used to remove unwanted residue e.g. the unreacted DMS oligomers.

In the examples above, a well array structure 2 is attached to a substrate 20 having electrodes 11. The substrate can include one of: a printed circuit board; plastic interposer made from a laser ablated plastic sheet; silicon interposer; and the surface of an application specific integrated circuit (ASIC) having exposed terminals on one face thereof to function as the electrodes. The substrate 20 can be a plastic sheet that has a well array structure laser ablated into the surface on one side. The plastic sheet can be coated with a metal film.

The well array structure 2 can be formed of a single component, which can include one of: a moulded material, such as a moulded polymer 102; a three-dimensionally printed object; or a patterned photoresist material. A three-dimensionally printed object can be made from materials including dielectric ink (UV Curable Acrylate), pseudo-polyimide inks, polyetherketone, polylactic acid, dielectric ceramics e.g. an aqueous solution of Li2MoO4 and ceramic particles, polyetherimide and polyimide.

Alternatively, the well array structure 2 can include two separate layers. That is, the well array structure can have a base layer that forms the well layer 2 e.g. cured mouldant 104 and an overhang layer 5. The overhang layer 5, therefore, can be said to extend at another level or layer from the well array structure 2.

The base layer 2 can be configured as a permanent layer in the device 100, and the support layer 5 that defines the overhangs can be removably attached therefrom to enable the device to be processed for re-use or re-positioning.

The base layer 2 defining the wells 4 can include at least one of: a moulded polymer 102; a three-dimensionally printed layer; plastic interposer made from a laser ablated plastic sheet; a patterned photoresist material; glass; silicon or silicon dioxide. The base layer has an array of walls 3 defining through-holes with apertures for defining the wells 4 when connected to the substrate 20.

The support layer 5 defining the overhangs can include at least one of: a moulded polymer 102; a three-dimensionally printed layer; plastic interposer made from a laser ablated plastic sheet; a patterned photoresist material; glass; or silicon dioxide. The support layer has pillars in the form of protrusions 51, 52 that support the retention of oil for forming amphiphilic membranes across the wells 4. Alternatively, the support layer can comprise solid-state nanopores or hybrid nanopores (biological nanopores located in solid-state apertures).

In light of the teaching herein, the methods of fabrication and examples below, various combinations of substrate 20, base layer 2 defining a well array and support layer 5 defining overhangs are configurable.

Figure 13 shows an example of a nanopore support structure 1 that can be used to make a nanopore sensing device 30, or portion thereof, for supporting a plurality of nanopores upon an array of wells 4 - only three of which are shown. The sensing device has a substrate 20 having a surface 10 upon which an array of electrodes 11 are located. The electrodes 11 are exposed at apertures or open-areas 158 at the bottom of the wells, as viewed. The electrodes can be connected to, or be configured upon, an electronic circuit. Upon the substrate 20 is a well array structure 2 having an array of walls 3 defining through-holes 160 with the apertures 158 for defining the wells 4 when connected to the substrate 20. The well array structure can be connected directly to the substrate 20, although this connection has been shown with adhesive layer 162. Additionally, or alternatively, the connection can use an adhesive surface or other such bonding materials or methods. When connected to the substrate 20 the through-holes that define the wells 4 and apertures 158 are aligned with the array of electrodes 11 such that the base of the wells of said well array structure, are defined, at least in part, by the electrodes. The base of the wells can also be defined, at least in part, by the surface 10 of the substrate 20.

To form the sensing device 30, before supporting a plurality of nanopores upon the array of wells, the substrate 20 can be is provided. This is to say that no further processing or manufacturing steps are required to the substrate, or well array structure, after it is connected to the well array structure that will negatively impact the performance of the sensing device 30.

The well array structure 2 is also provided separately, having pillars 164 on one side and apertures 158 on the other. By providing the substrate and well array structure separately they can be manufactured without the conditions in which they are manufactured having any detriment upon the other component.

The substrate 20 can have an electronic circuit on the surface 10, or the substrate can be a printed circuit board. The electronic circuit and electrodes can be provided on the surface of an application specific integrated circuit (ASIC).

The separate provision of the well array structure can include forming the well array structure 2 on the surface of the substrate 10 i.e. after the substrate 20 has been provided the surface 10 can be used as a base upon which the well array structure is formed. The well array structure can be moulded and then applied to the substrate or can be moulded directly onto the substrate surface 10.

The electrodes 11 are shown in Figure 13 as part of the surface 10 of the substrate 202. In an alternative example, the substrate can be provided with through-holes and filled with a conductive material to provide a conductive via and the exposed portion of the via functions as an electrode forming, at least in part, a base of a well 4 in the aperture 158 of the well array structure.

The well array structure can have a thickness of between 10 microns and 1000 microns, and preferably between 10 microns and 500 microns, and more preferably between 100 microns and 500 microns. The depth of the wells can be between about 5 microns and about 1500 microns, and preferably between 10 microns and 500 microns, and more preferably between 100 microns and 500 microns. The pitch between the wells in the well array structure can be between about 10 and about 1000 microns, and preferably between 10 microns and 500 microns, and more preferably between 20 microns and 100 microns. Through-holes can have a diameter of less than 200 microns, and preferably between 10 microns 200 microns

After the sensing device 30 is formed the wells 4 can be populated with liquid and membranes formed across the wells before inserting nanopores in the membrane. The membranes can include (i) amphiphilic membranes, and support biological nanopores, (ii) solid-state membranes, and have solid-state nanopores, (iii) solid state membranes and support a biological nanopore in a hole within said solid-state membrane.

The sensing device 30 can be fabricated in a number of ways. The function of the sensing device can also be implemented by incorporating a mixture of the different modular components described herein - such as the substrate 20 and well array structure 2, which can be fabricated themselves in different ways using different materials. Overall, however, the important elements i.e. the substrate 20 and the well array 2 are connected together in the later stages of fabricating the sensor. In many of the examples herein the well array structure 2 can be removably attached to a substrate such that it can be removed and replaced with a new substrate, thus making the sensing device recyclable. In one example, forming the sensing device 30 includes forming the well array structure 2 having the through-holes 160 and further providing a patterned adhesive surface, which can be an adhesive layer 162, on at least one of the well array structure 2 or the substrate 20, wherein said patterned adhesive surface defines a plurality of portions without adhesive.

The portions without adhesive can be areas such as holes or voids in the patterned adhesive surface that expose the electrodes 11 to the through-holes in the well array structure. The patterned adhesive surface can also be one of the surface 10 of the well array structure 2 or substrate 20 that have been prepare or treated to give the connecting surface adhesive properties, for example by exposing the surface to UV light or plasma treatment.

The well array structure 2 is aligned and connected to the substrate 20 using the adhesive layer 162 such that the array of electrodes 6 located on a surface thereof define, at least in part, a portion of a single well.

The substrate 20 supports or incorporates an electronic circuit having electrodes 11 and is formed independently of said well array structure which contacts or comprises said electrodes. The adhesive surface of the adhesive layer 162 can be heated to assist adhering the well array structure to the substrate. The heating can be limited to a temperature below the outgassing temperature of components of the electronic circuit. Electromagnetic radiation can, additionally or alternatively, be used to adhere the well array structure to the substrate 20. The adhesive layer 162 may be a photo-patternable adhesive that is exposed to light prior to adhesion. Additionally or alternatively, the well array structures and/or electrodes located at the base of the wells can be exposed to a plasma treatment that functions to at least one of: clean exposed surface and temporarily change the properties of exposed surfaces to become adhesive in nature e.g. sticky.

As shown in Figure 13, the adhesive layer 162 can be an independent layer to the well array structure or the substrate. It can, however, be integrated into one of the well array structure or the substrate. Or it can be a region of the well array and/or the substrate that has been modified to have adhesive properties, such as by heating.

One example method of the invention allows the well array structure 2 to be fabricated separately from the substrate 20 and then for them to be combined after they have been optimally processed. The method is composed of (i) making a stand-alone microfluidic well arrays structure 2 from thick film photoresist about 120 microns thick, which is available from TOKYO OHKA KOGYO CO., LTD. (TOK), (ii) making a stand-alone patterned adhesive layer 162 with at least one of photo-definable adhesives (also available from TOK), laser cut adhesives or liquid adhesive and controlled wetting of the substrate/resist interface, or preparing the surface of the well array structure or the substrate to have adhesive properties (iii) alignment and placement of the adhesive on to the substrate 20, e.g. by robotic placement, (iv) alignment and placement of the microfluidic structure 2, e.g. by robotic placement, and (v) post-processing to cross link the materials using, for example, UV exposure or heating.

In more detail, the photoresist can take the form of thick film laminates that are laminated onto a sacrificial layer. The sacrificial layer can, for example, be Omnicoat (RTM) or polystyrene, which can be spun onto a Si wafer. The use of the silicon layer is just one technique that enables the well array structure to be fabricated independently of the substrate to which connects. As described herein, making the components separately avoids a manufacturing process of one component adversely influencing the other.

To reduce sticking to the mask during the lithography step either a post-process heating process is adopted or the mask can be treated beforehand to avoid sticking.

To minimise stress in the laminate occurring because of the connection with other surfaces post-processing temperature control is adopted.

When the adhesive layer is made from photo-definable adhesives a plurality of vias are provided, which align to the apertures 158 in the microfluidic structure such that they function to extend the through-holes 160. The adhesive layer 162 can be selected to have the same surface energy as the well array structure 2. By way of example it can be obtained from TOK (NC-00075S; thicknesses of 20um and 50um).

Other methods of forming stand-alone patterned adhesive layers include (but are not limited to): using a laser machine to ablate appropriate vias in pre-existing adhesive materials such as pressure sensitive adhesive layers (PSA), using liquid adhesive and controlled wetting at the interface of well array structure and substrate, using an aerosol spray or micro-drop dispenser, using capillary action with a liquid adhesive. The adhesive material can have the same surface energy as the well array structure.

Ensuring complete cross-linking can be achieved by baking. The temperature of the bake is limited to be below the outgas temperature of the substrate 20, which can be polymers present on a PCB for example.

After independent formation of the well array structure 2 on, for example, a Si wafer it is removed. The removal step can include a toluene lift-off before attachment to the substrate using adhesion.

The adhesive layer can be an adhesive surface i.e. the well array structure has adhesive properties on the surface adjacent the apertures 158. This can be achieved by not hard-baking the well array structure and leaving residual solvents and/or uncross-linked material. Heat and pressure can be used to enable adhesion.

The well array structure can have a thin film of adhesive transferred on to its surface in a stamp-transfer process. The stamp can be made of polydimethylsiloxane (PDMS). The stamp can have fine bristles to control transfer volumes of adhesive. The surface energy of PDMS is suited to controlling the volume of adhesive pinned to the bristle tops because of its low surface energy. The stamp-transfer is preferentially made to the well array structure 2. This is because the substrate can have electrodes thereon and while the layout and density of the bristles can be arranged to inhibit any transfer of adhesive to the electrode surface on the substrate 20 the application of adhesive to the well array structure mitigates this happening.

In another example, not according to the claimed invention, forming the sensing device 30 includes forming the well array structure 2 using a moulding technique. This can achieve independently of the manufacture of the substrate 20. The independent formation of the well array structure can occur either on a reference surface before subsequent removal and application to the substrate or, alternatively, the moulding can take place directly upon the substrate.

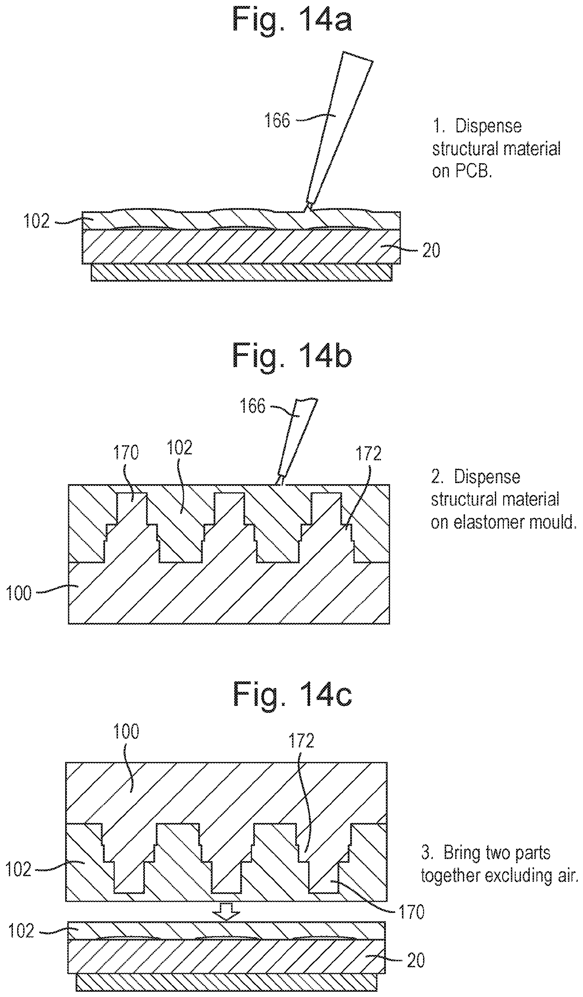

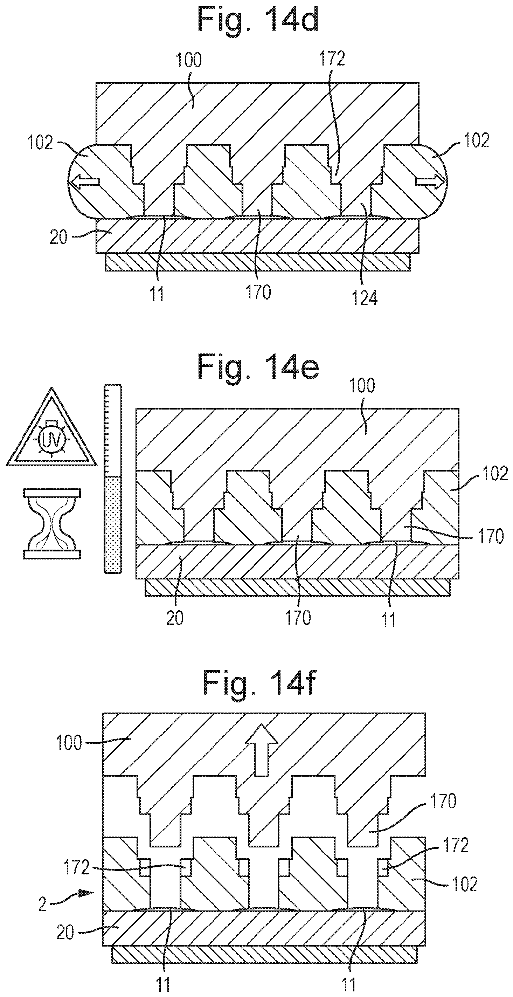

The basic steps of a moulding example are illustrated in Figures 14(a) to 14(f). Initially, a dispenser 166 deposits a mouldant 102, referred to hereinafter as a polymer 102, by way of example, on the surface of a substrate 20 having electrodes 11. The polymer 102 is distributed evenly over the surface of the substrate.

Separately, in a second step, polymer 102 is dispensed into a mould 100, wherein the mould is a negative of the well array structure 2 to be formed. The mould 100 can be fabricated from a master 168, wherein said master is identical to the well array structure to be formed. In the process of researching and developing the inventors choose to use the well array structure of a current MinION (RTM), although the examples of the invention are not limited thereto. Alternatively, the mould 100 can be fabricated from scratch without a master. In this way imperfections can be mitigated when the mould is created.

In a third step, as shown in Figure 14(c), the mould 100 having been filled with polymer 102 and evenly covered, such that no portion of the mould is exposed, is aligned with the substrate 20 covered with polymer 102. The alignment brings the mould 100 and substrate 20 together such that protrusions 170 align and correspond to the electrodes 11.

In a fourth step, when the mould 100 and substrate 20 are brought together under pressure the protrusions 170 squeeze excess polymer from the surface of the electrode. The elastomeric properties of the mould 100 allow it to conform and accommodate variations in the surface of the substrate 20, such as dome-shaped electrodes, uneven surface features or curvature of the surface of the substrate 20. In a fifth step, the polymer 102 is cured before the mould is separated from the substrate, in a sixth step, leaving the polymer attached to the substrate 20. The polymer has become the well array structure 2.

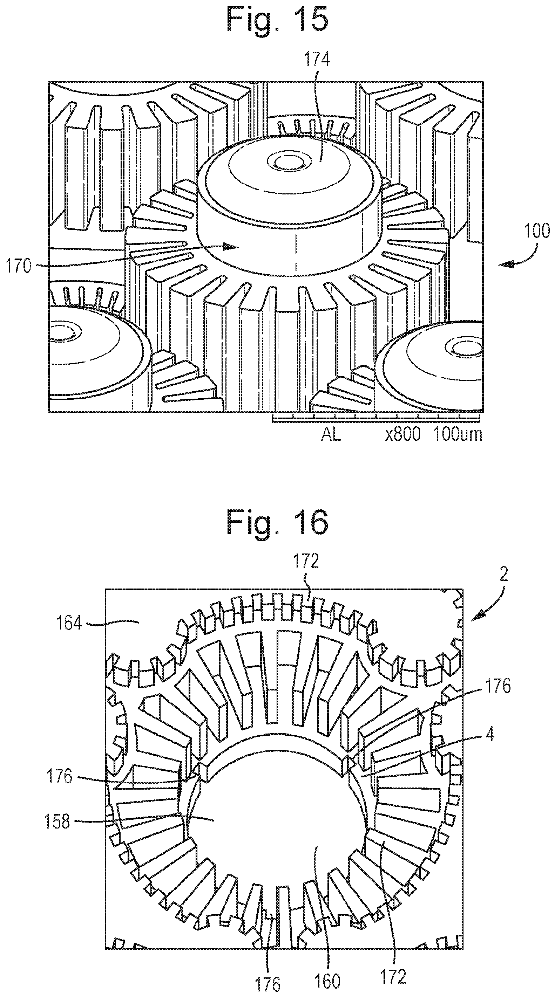

Figures 15 and 16 show a mould 100 and the resulting polymer 102 shape it can produce. The protrusion 170 is shown having fins 172 and an extension 174 that clears polymer from the electrode during formation of the polymer. The purpose of the well array structure 2 is clearly documented in WO2014/064443. What cannot be appreciated from the known art is the moulding techniques and adaptations of the hardware required to optimise the manufacturing process and subsequent performance of a nanopore sensing device having an improved well array structure. Figure 16 shows features that can control the distribution of pre-treatment oil prior to the formation of membranes over the well array structure, such as three recesses 176. In this example the recesses are shown as rectangular notches or grooves. They can be evenly spaced but could be any number or arrangement of recesses or pockets for retaining a pre-treatment oil or the like. The recesses function to inhibit oil pooling at the bottom of the compartment 4 and covering an electrode 11.

The protrusion 170 functions to form the through-hole of each compartment 4 and displace polymer from the electrode 11. Partial fouling of flat electrodes can occur and result in a film of moulded material remaining covering a portion of the electrode surface. To counter this, the protrusion 170 is provided with an extension that can be curved or domed at the end of the protrusion, as shown in Figure 15, or a stepped extension of progressively smaller discs. The purpose of these extensions 174 at the end of the protrusion on the mould 100 is to generate a pressure gradient on the polymer, which is a liquid moulding material, as the elastomer of the mould is compressed. This functions to extruding the liquid as the mould conforms to the electrode surface and contacts the electrode. In Figure 15, a dimple resides in the centre due to a photolithography defect. This defect turned out to allow mouldant to accumulate in the dimple while clearing surrounding areas of the electrode. One or more dimples can be provided on the top of the protrusion for the purpose of allowing mouldant to accumulate in the dimple. The dimple can, for example, be star-shaped e.g. a 5-sided star.

Alternatively, the mould 100 can be placed against the substrate and a mouldant 102 can be injected between the mould and substrate before being processed to cure and secure the mouldant to the substrate. The mould could then be removed to leave the well array structure upon the substrate.

The mouldant 102 has been described as a polymer such as poly(methyl methacrylate). The polymer can be dissolved in a solvent before being deposited onto the underside of the mould 100. For example, PS (35KDa) can be dissolved in 500mg/mL of chloroform. After moulding the polymer can be heated to evaporate the solvent before removing the mould from the substrate.

The amount of solvent in the mouldant can be selected to inhibit any change in the dimensions of the mould 100, when susceptible to absorbing some of the solvent and losing a degree of structural rigidity.

The mould can be a thermoplastic, or it can also be made of a silicone material, such as polydimethylsiloxane (PDMS). The master 168 can be made from a robust dimensionally stable material, such as silicon wafer.

The mouldant can be a two-component thermal cure epoxy or UV curable material. Thermal cure epoxy was found to be slower to cure than UV curable epoxy and require the mould to be held in place for a longer period of time.

Mouldant materials can include one or more of epoxy, acrylamide/methacrylamide, polyester, styrene copolymer, vinyl, polyurethanes (isocyanate/amine chemistry) or polyimide.

Numerous mouldant materials are available for various manufacturers, and include: DYMAX (RTM) materials, including part 6-621, 431, 3069, 3069-GEL, 3099 and 1072-M; LOCTITE materials, including part AA 3321 LC, AA 3936, 3922, 3921, 3311, 3301 and 3211; EPOXIES materials, including part 7108, 7156 and 7159; NORLAND PRODUCTS INCORPORATED materials, including part NOA81, NOA61, NOA88 and NOA 83H; MasterBond materials, including part UV10MED, UV22DC80-1MED, UV10, UV10TKLO-2, UV16 and UV15LV; Epoxy Technology materials, including part OG198-54, MED-OG198-54, OG142-87 and OG-112; Permabond materials, including part 4UV80 and UV605; and Ostemer materials, including part '322'. By way of example, two suitable materials were from Masterbond (RTM) part UN10MED and EpoTek (RTM) part OG198-54. The connection between the well array structure 2 and the substrate 20 can be semi-permanent. In other words, they can be peeled apart such that a formed well array structure can be removed and reapplied to another substrate. Or, after removal, a new well array structure can be formed in its place. In this way the substrate and associated devices or components can be recycled.

Although the above processes describe forming or attaching a well array structure to a substrate such that the electrodes are accessible through the through-holes, the electrodes 11 can be subjected to further processing to clean their surface. The cleaning can comprise at least one of chemical cleaning or plasma treatment.

The mould 100 can be independently formed on a known TSV 4, which would minimise the effects of processing on the surface energy of a well array structure 2, or the mould can be independently formed on a substrate such as a printed circuit board.

While well arrays can be moulded with fine features for oil control, such as those shown in Figure 16, the shape of the well 4 can be formed like those shown in Figures 1 to 4 i.e. a well 4 having featureless walls 3 can be formed adjacent an electrode 11, while an upper overhang 5 layer can be formed having protrusions 51, 52 analogous to the fins 172. The moulding examples herein show that wells can be moulded with features having high-aspect ratios in the region of 20:1, and complex structures, such as those shown in Figure 16. It follows that moulding can create high aspect ratio wells and/or an overhang 5 having lower aspect ratios, in the region of 3:1 to 2:1.

In a further example of the invention an improved substrate 20 can use alternative materials and manufacturing methods to those described above. This can enable a cost-efficient consumable, high throughput and low material cost sensor to be fabricated. This example provides an integrated electrode using laser machining and screen-printing methods. The method is compatible with reel to reel or panel to panel processing.

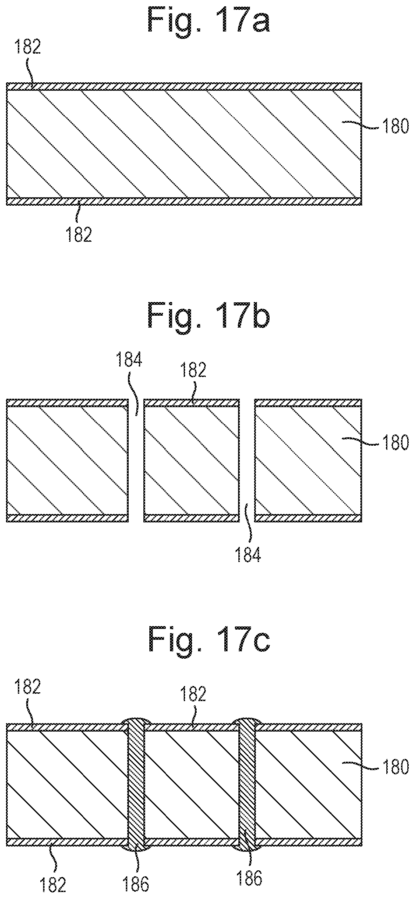

By way of example, Figures 17(a) to (e) illustrate progressive steps that can be taken to provide a substrate 20 with electrodes 11 upon which a well array structure 2 can be formed. The method involves taking a sheet 180 of thermoplastic foil that can be, for example, polyimide, polyetherimide, polyether- ether- ketone (PEEK), polycarbonate or polyethylene terephthalate (PET). The thickness is about 0.5mm or less. A metal film 182 is formed on the top and bottom side, as viewed, using a metallisation process. The metallisation process can be sputtering, and the metal applied can be, for example, silver, gold or platinum. The metallisation provides for re-distribution lines (RDLs) to be formed on the surface of the sheet.

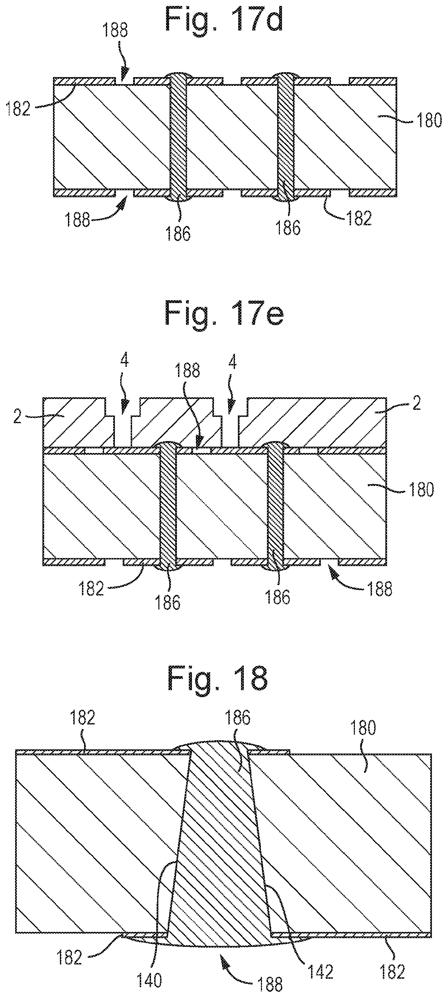

Figure 17(a) shows such a sheet 180 having a metal film 182. A laser is used to machine a plurality of vias 184 through the metalised film, as shown in Figure 17(b). The vias 184 are then filled with a conductive paste, such as carbon or silver paste, before being cured to provide conductive vias 186. Each conductive via 186 has a low resistance of between about 200 and about 300 ohms. The paste can be filled using screen-printing. When screen printed the contact between the conductive via 186 and the metal film 182 is increased by the formation of domed mushroom-like areas of excessive paste at the via 184 openings, as shown in Figure 17(c). This overflow can function to provide a connection between the metal film on each side of the sheet 180. Thereafter, and RDL pattern is formed on the metal film 182 on the top and bottom surfaces to provide a circuit having a feature 188 such as a contact track 188, tracks 188 or contact pad 188, including electrodes 11, on one or both sides, as shown in Figure 17(d). Figure 17(e) shows how a portion of the metal film 142 can be retained to define the electrode 11 and close the aperture 158 and define the bottom of a compartment 4 of a well array structure 2.

The well array structure 2, as described above, can be formed independently using (i) a photoresist layer, with or without a separate adhesive layer, or (ii) a mould 100. The use of a mould accommodates the uneven surface caused by the tracks 188 and/or the domed ends of the conductive via 186 and is particularly suited to accommodating variations in height on the surface of a substrate 20.

To implement the metal film 182, a sheet 180 can be formed by sputtering metal on to the sheet before subsequently using laser ablation to define electrodes 11 and any circuitry. By way of example, to provide a high purity electrode 11 platinum can be sputtered on to a PEEK sheet. A high-quality electrode can be provided to maximise electron transfer when the sensor device 30 is operating.

Figure 18 illustrates the impact of the shape of the via 184 upon the dimensions of the interface with the electrode, or other circuitry, of the metal film 182. It is clear that the size of the aperture of the via formed at the surface of the sheet 180 by laser drilling influences the size of the conductive via contact area. The vias formed in Figure 17(b) have parallel sides and high-aspect ratios that create small apertures in the surface and, subsequently, have minimal exposure on the surface of the sheet 180. When filled with screen-printing small dome-shaped contacts are formed. In practice, the shape of the via 184 is determined by the thickness of the sheet 180 and the drill geometry i.e. the laser drilling process. To maximise the utilisation of the surface of the sheet 180 the footprint of the via 184 and conductive via 186 should be minimised.

As an alternative to coating a sheet 180 with metalised film 182, drilling and then filling the formed via 184 with conductive paste a bare sheet without a metalised surface can be drilled first and then coated using physical vapour deposition (PVD) process. A PVD metal coating can be formed to extend from the planar surfaces of the sheet and in to the via 184 thus forming an alternative conductive via 186 to the one shown in Figure 18.

In practice, a high-aspect ratio via 184 extending perpendicularly from one side of the sheet to the other, as shown in Figure 17(b) has a very small aperture on the surface and a taper angle of 90 degrees. Consequently, PVD coating the walls of the via furthest from the surface of the sheet is difficult because access is restricted.

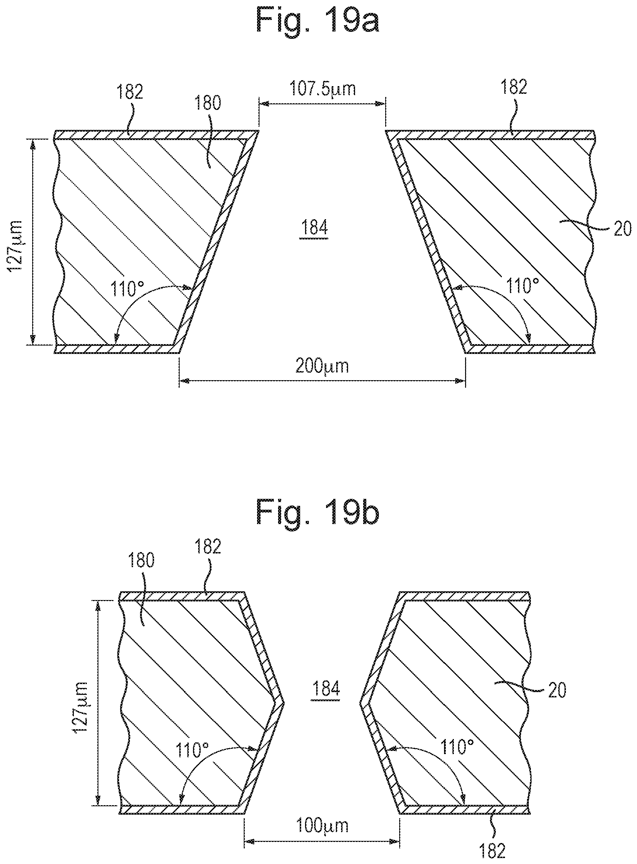

Figure 19(a) shows the dimensions of a sheet drilled from one side and the metal coating 182 coating that forms a via with flat sides, while Figure 19(b) shows a sheet drilled from both sides, and the subsequently applied PVD coating 182 that forms a via with ridged or hipped sides. The angle between the planar surface of the sheet and the side of the via extending therefrom is the taper angle. Therefore, the taper angle at one or both sides is greater than 90 degrees.

When the taper angle is greater than 90 degrees on only one side of the sheet 180, as shown in Figure 19(a), one opening of the via is greater in size than the other. In this case the ratio of aperture size or diameter of one side to the other can be about 1:4, or preferably about 1:3 and more preferably about 1:2. In this arrangement one side has an aperture receptive to PVD such that the walls of the via are coated all the way through.

Drilling from both sides results in the taper angle being greater than 90 degrees on both sides of the sheet 180, as shown in Figure 19(b). The openings can have different sizes, although the via can be substantially symmetrical - appearing to have two lines of symmetry, one horizontal and one vertical, as viewed. In this case the ratio between the aperture size or diameter on one side compared to the opposite side, can be about 1:1. This minimises the footprint of the via as viewed from the surface of the sheet, and as viewed from both sides. Moreover, in the arrangement of Figure 19(b) both sides of the via are receptive to PVD such that the walls of the via are coated all the way through.

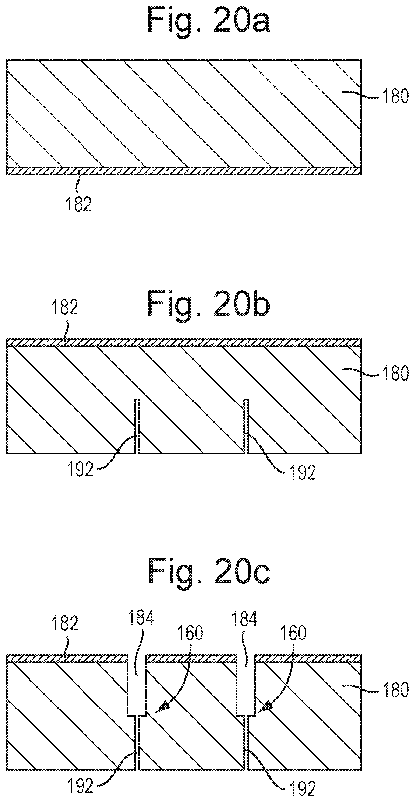

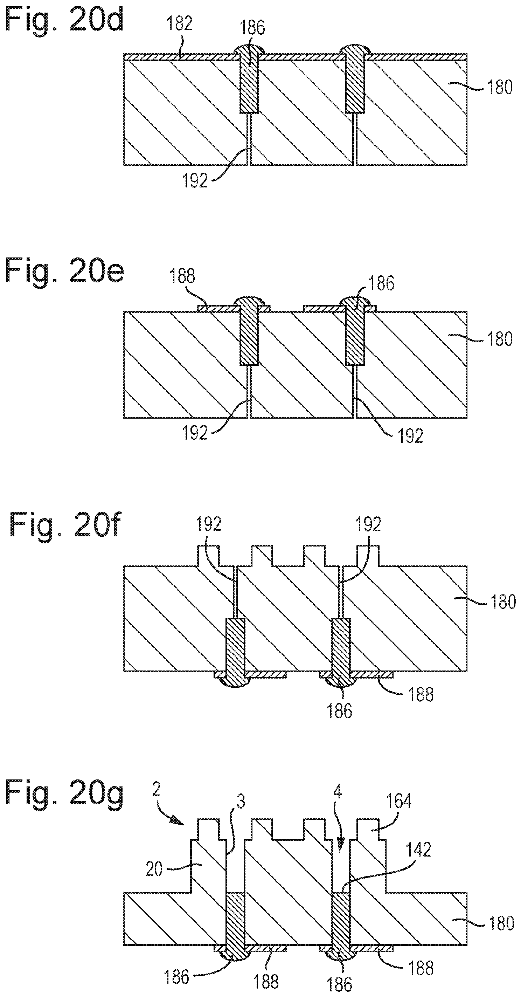

Figures 20(a) to (g) show fabrication steps for a hybrid well array 190 that incorporates the function of the substrate 20 and well array structure 2, described above, into a single-piece component for nanopore sensing. Like reference numerals refer to like features. The fabrication of the hybrid well array 190 begins with a sheet 180 having a metal film 182 formed only on one side, or having a metal film deposited only on one side, to provide a sheet as shown in Figure 20(a). Vent holes 192 are drilled on the opposite side of the sheet to the metal film, as shown in Figure 20(b) before a partial via 184 is drilled through the metal film on the opposite side of the sheet 180 to the vent 192. The resulting structure can be seen in Figure 20(c) having a via 184 extending from one side of the sheet to the other, said via having a wide drilled portion and the narrow vent 192. In Figures 20(d) and 20(e) the wide end of the via 184 is filled with a conductive paste before the metal film is trimmed to form a circuit connected to the conductive vias 186. It is to be noted that the vent is too narrow to fill, or fill completely, with the conductive paste. The purpose of the vent is to allow the wide portion of the via to fill completely by inhibiting trapped air from preventing the via being filled. Thereafter, the vent 192 side of the sheet 180 is processed to form pillars 164 as shown in Figure 20(f) before material around those pillars is removed to define walls 3 of compartments 4 and expose the conductive via 186 as shown in Figure 20(g).

The properties of the electrodes 11 on the substrate 20 influence the performance of the sensing device 30. As described above, the sheet 180 can be sputtered with metal and subsequently laser ablated. Optimum performance has been achieved with sputtered platinum upon a poly(etheretherketone) PEEK sheet. Vias 184 can be subsequently filled. The base of the compartment 4 can be defined by the sputtered metal film 182 that is connected to a conductive via 186, as shown by way of example in Figure 17(e). The conductive vias 186 are configured to provide low resistance electrical contacts with resistance values for between 150ohms and 300ohms, or between 200ohms and 300ohms.

The material selected for the sheet 180 was PEEK. In the examples, the sheet had a dual gloss finish and a thickness of 0.25mm. PEEK was chosen based on at least one of a number of factors, including: mechanical stability to provide reliable connection with a connector, such as Samtec z-array connector (https://www.samtec.com/connectors/highspeed-board-to-board/high-density-arrays/zray); the PEEK had good adhesion properties with a sputtered platinum layer allowing high resolution track and gaps to be formed without flaking; and PEEK is suitable for achieving high aspect ratio laser drilled via holes. Other materials found to be suitable with comparable performance include Polyetherimide (PEI).

Before sputtering, the sheet 180 is cleaned with, for example, ethanol before loading into a sputtering chamber. In the examples, 50nm of platinum was coated on both sides of the film.

A laser was used to pattern and drill the metalised sheet 180. The pattern laser can be, for example, a SMI laser (MSV-301). The drilling laser can be, for example, a direct write laser (MSV-101 UV).

The vias 184 were filled was carried out using a DEK^{™} screen print process. Silver ink was used to fill the vias from the backside before flipping the panel and capping the vias on the frontside. The ink was then cured in the oven at 110°C.

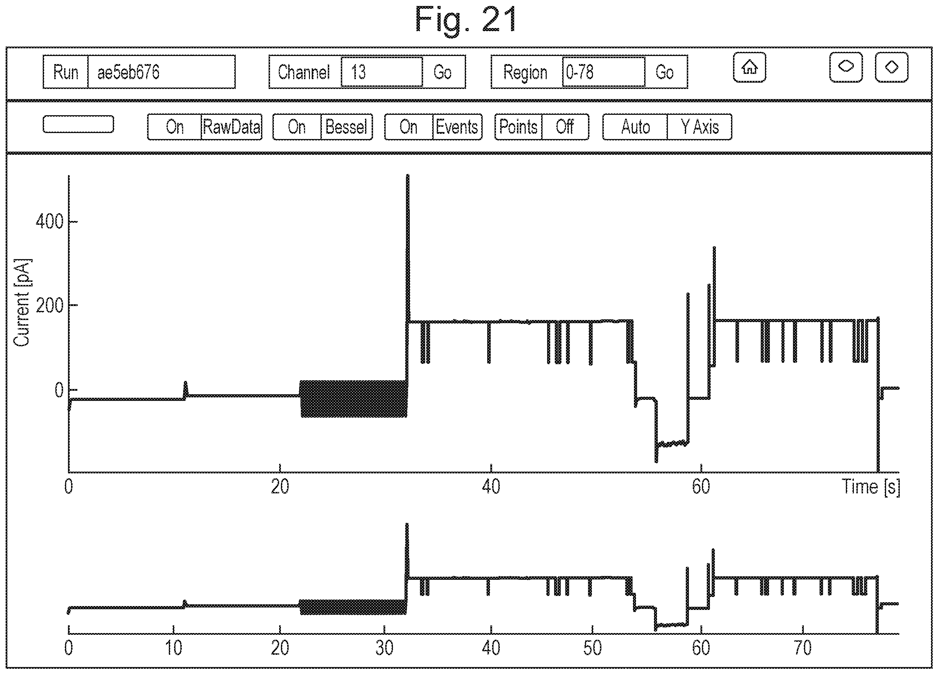

In each of the examples the performance of the sensing device 30 was evaluated by manufacturing a known MinION (RTM) device with the combined well array structure 2 and substrate 20, or hybrid well array 190, in place of known structures and wafers. After assembly, the well array structures were fluid filled and the membranes formed across the compartments 2 were populated with biological nanopores. During the evaluation, indicators of adequate performance include: an open pore current between about 180 to 200pA with 180mV applied across the pore; noise levels of between about 2 to 3pA standard deviation on the open pore current; and sharp thrombin binding aptamer (TBA) events indicating no additional high RC time constants in the setup. Details of TBA use is described in WO2014/064443. An ion channel recording with TBA on each of the examples demonstrated results like those shown in Figure 21, which shows the ionic current measured through the pore being blocked by an analyte (TBA), the pore being inserted in a membrane supported on a well array structure 2 made with photoresist upon a substrate 20 made with a plastic sheet 180, as described above.

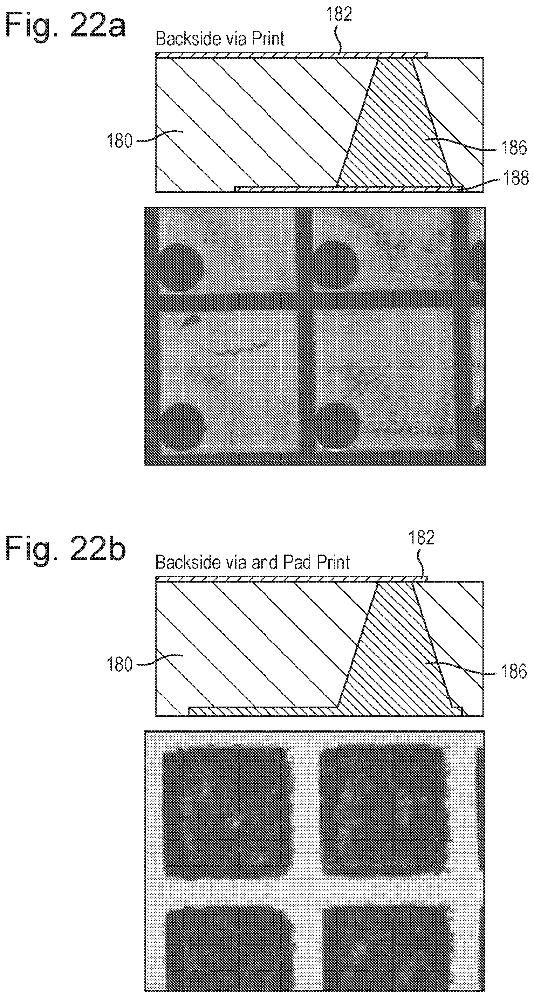

A number of examples have been described as having a sheet 180 with a metal film 182 on both sides, with tracks and/or contact pads formed in the or each metal film. As a lower cost alternative the sheet can be coated with a metal film on one side, while contact pads are screen printed on the non-metalised side, thus no metal patterning is required. The vias 140 can be filled and the contact pads formed in a single step. Figure 22(a) shows a schematic of a sheet having a metal film on both sides, with a conductive via 186 therethrough connected to a contact pad 188. The surface of both metal films 182 have been trimmed - this schematic is shown above an image of an actual pad. In the example of Figure 22(a) the metal films are platinum.

Figure 22(b) is a schematic of a sheet having a metal film on one side, and when the conductive via 186 is screen printed on the sheet 180 the contact pad 188 is formed at the same time. The conductive via 186 and contact pad 188 both have the same material, which in this example is silver.

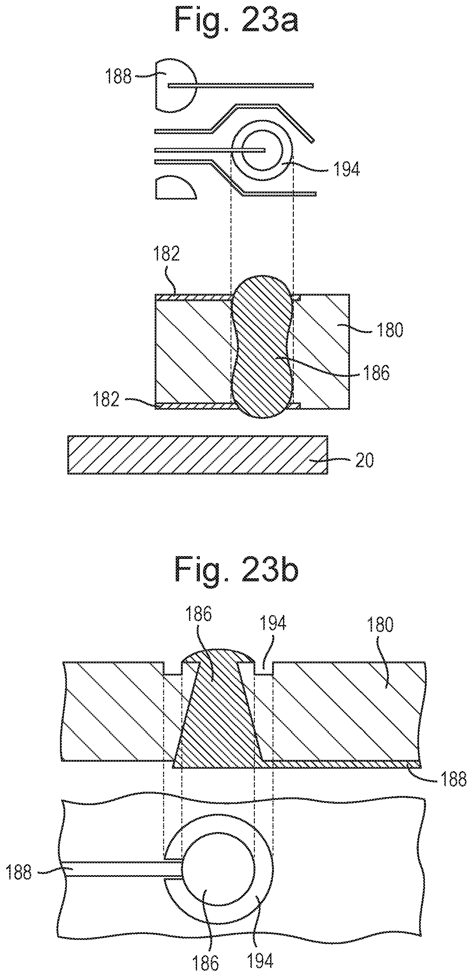

The density of vias 184, tracks 188 or contact pads 188 formed during screen printing processes is influenced by the controlled spread of paste. The channel density is defined by a combination of the track or pad size, the gaps between and the print size. Preventing spread of the ink during printing is important to tightly define print size. This also inhibits short circuits by controlling the deposition and spread of conductive paste. A capillary stop 194 can be formed adjacent a via 184 to inhibit screen printed ink from spreading. A plan view of a conductive via 186 adjacent a track is shown in Figure 23(a) aligned with a sectional elevation view of a sheet 180 with the conductive via 186 and beneath the sheet a PCB. The diameter of the conductive via 186 forming an electrode is 100 microns, while the capillary stop is shown extending around the aperture created by the via 184, which limits the print diameter to 110 microns. Figure 23(b) shows the geometry of the capillary stops 194 in more detail. A groove is formed adjacent but separated from the aperture of the via 184 in the sheet 180. The level of the aperture is in the same plane as the sheet 180, and the groove extends into the sheet. In the example shown the groove does not enclose the via to allow a track to extend along the planar surface of the sheet 180. The groove of the capillary stop is an effective way of providing pinning points to prevent ink spreading.

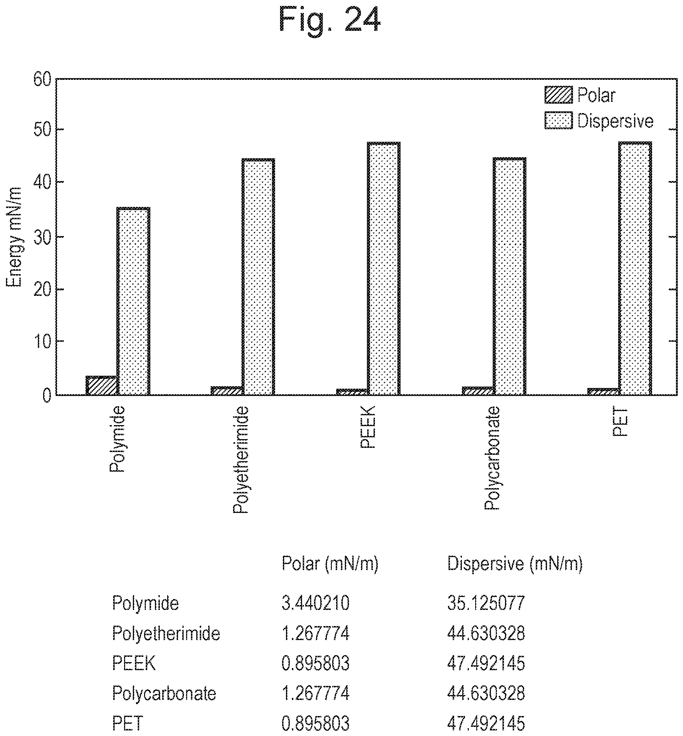

The material selected for the sheet 180 took in to account its application as a well array structure in a nanopore sensing device 30. The values required of a dispersive component of the sheet material is 40-55mN/m and the polar component should be less than <3mN/m. Contact values of PEI, PEEK, PC and PET were assessed and their properties tabled, as shown in Figure 24. During the evaluation of these materials their native surface properties through the laser ablation process is also key. These surface properties can be tuned based on atmosphere during the ablation steps minimising oxidation.

To obtain the required surface energy while not relying on maintaining the bulk properties the process can involve: functional groups remaining at the surface post ablation, which can be modified to achieve the required surface properties (silanes); and surface coating using plasma treatments / thin film depositions.

As described in the devices and methods of forming the devices above, the sensing device 30 can have a well array structure 2 attached to a substrate 20 having electrodes 11. The well array structure 2 can be formed of a single component. Alternatively, the well array structure 2 can include two separate layers i.e. a base layer that forms the well layer 2 and an overhang layer 5.

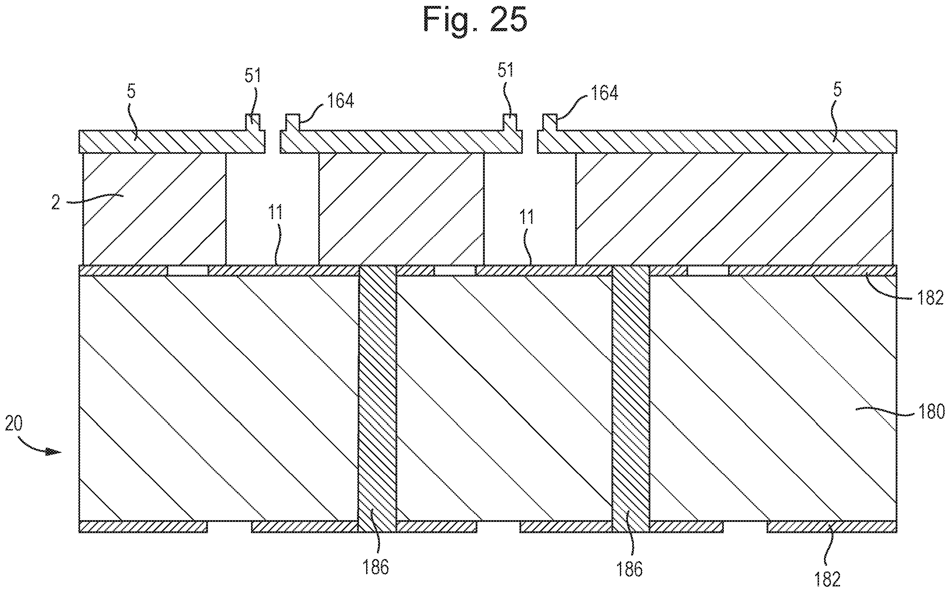

An example of a sensing device 30 incorporating a combination of layers fabricated using different techniques, such as those described above, is shown in Figure 25.

Figure 25 includes a substrate 20 formed using a sheet 180 having metal film 182 that has been laser etched to have vias 184 and a patterned surface defining electrodes 11. The electrodes connect to the opposite side of the substrate through conductive vias 186 to contact pads 188. The sheet and metal film could, alternatively, be implemented by a printed circuit board, or be provided on the surface of an ASIC device having exposed electrodes. A well array structure 2 is formed upon the substrate 20 having wells 4 defined by walls and located over the electrodes 11. The well array structure can be formed from 3D printing, moulding, laser etched plastic or an etched laminate sheet.

The well array structure 2 and the electrodes 11 can be plasma cleaned prior to the attachment of the overhang 5 layer, which is provided upon the well array structure 2, and similarly, can be formed from 3D printing, moulding, laser etched plastic or an etched laminate sheet. The overhang layer 5 can be removably attached to the well array structure 2. Protrusions 51 are shown on the overhangs external to the well and could similarly be applied to the other surfaces of the overhang i.e. facing the electrode and/or extending in the plane defined by the overhang layer.

The techniques used to make the well array structure 2 and overhang layer can be made from different techniques taught herein. By way of example, the well array structure 2 has high aspect ratio wells formed from a moulding. The aspect ratio of the wells is defined by the depth and width of the wells, which is influenced by the well array structure material 2, and can be upwards of about 3:1. The overhang layer can also be formed from a mould, or alternatively a laser ablated plastic sheet 180. The aspect ratio between the thickness of the film and the height of the protrusions 51, 52 is lower than that of the wells, and can be as low as about 2:1.

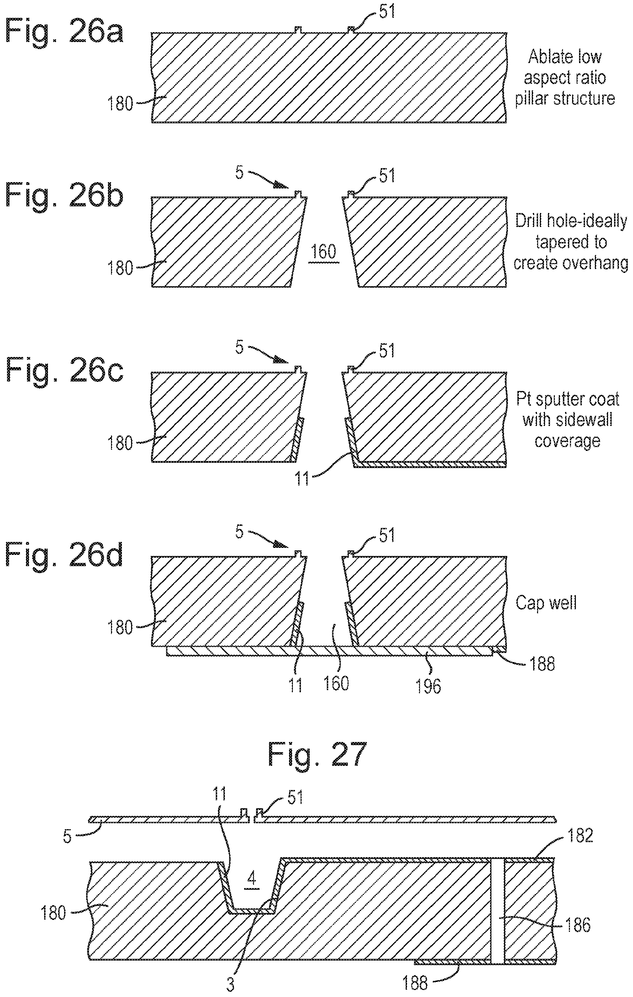

Figures 26(a) to (d) are steps taken to fabricate an overhang in a single bare sheet 180, wherein low aspect ratio protrusions, which can be upwards of about 2:1, can be laser formed upon an upper surface, as viewed, before a hole is drilled through the sheet to create walls 3 that define wells 4. It is to be noted that the hole is drilled to form tapered walls as described in relation to Figure 19 above, such that overhangs are formed in a single layer. After the hole is drilled, surfaces of the walls 3 are coated, preferably with platinum sputtering, for form electrodes 11 and contact pads 188. The platinum coating can be trimmed using lasers. Finally, a closure 196 can be applied over the surface of the substrate 20 having the electrodes 11 and contact pads to define the base of a well 4. The closure can be a further substrate 20

Any electrode material can be applied, and platinum is proposed by way of example because it is particularly suitable for sensing purposes. Allowing the laser to pass straight through the sheet 180 improves the manufacturing process, because no electrode or other such structure inhibits performance of the laser drilling. The use of laser drilling is suited to the angular drilling. Laser drilling is also suitable for forming low aspect ratio features, such as the protrusions. Incorporating an overhang layer of material in a single part can simplify the manufacturing process. The post laser drilling hole through the sheet and subsequent electrode coating can result in an electrode having an increased surface area compared to an electrode at the base of a cylindrical well 4.

Figure 27 is a portion of a sensing device 30 formed on a substrate that can be a laser-etched sheet 180 or a PCB having a blind via 198 defining a well 4. The well can have tapered sides to increase the electrode 11 area in the well, and this also makes coverage easier. Laser drilling has no shape constraints and various shapes of well profiles can be formed. The substrate is processed with laser etching before filling vias 184, 186 and creating contact pads 186 on one side of the substrate 20. The well 4 is similarly coated, preferably with platinum, to define an electrode 11 in the well and the contact pads. The electrode extends from the well for connection to the via 186. The well is capped by an overhang 5 layer, shown above the substrate prior to attachment, having protrusions 51.

Alternative methods are also possible for forming the nanopore support structures 1. In some embodiments, the UV radiation is UVA radiation. In some embodiments, the UV radiation is provided by a UV laser (e.g. a gas laser, laser diode or solid-state laser). In some embodiments the UV radiation is provided using a UV lamp. In some embodiments the UV radiation is high intensity UV radiation. A combination of methods may also be used for different components. For example, moulding may be used for some components, and other components may be formed from photoresist material. Some components, such as the electrodes may be formed by etching, optionally chemical etching.

After making the nanopore support structure 1, for example using any of the above methods, the nanopore support structure 1 may be used to make a nanopore sensing device 30, for example as shown in Fig. 3. In this case, the nanopore support structure 1 is incorporated into a planar structure 40, and the nanopore sensing device 30 is assembled incorporating the planar structure 40.

Figs. 1 and 2 show the membranes 7 extending across the aperture 6 and the nanopores 8 inserted in the membranes 7, but some types of nanopore support structure 1 may be provided without the membranes 7 and nanopores 8. In that case, the end user of the nanopore support structure 1 carries out the steps to form the membranes 7 and cause the nanopores 8 to insert therein.

Examples of the membranes 7 and the nanopores 8 are as follows.

In one type of nanopore support structure 1, the nanopores 8 are biological nanopores and the membranes 7 are capable of having the biological nanopores 8 inserted therein.

The membrane 7 may be an amphiphilic layer, that is a layer formed from amphiphilic molecules, such as phospholipids, which have both hydrophilic and lipophilic properties. The amphiphilic molecules may be synthetic or naturally occurring. Non-naturally occurring amphiphiles and amphiphiles which form a monolayer are known in the art and include, for example, block copolymers (Gonzalez-Perez et al., Langmuir, 2009, 25, 10447-10450). The membrane 7 may be a triblock or diblock copolymer membrane.

The membrane 7 can be one of the membranes disclosed in WO2014/064443 or WO2014/064444. These documents also disclose suitable polymers.

The amphiphilic molecules may be chemically modified or functionalized to facilitate coupling of the polynucleotide.

The amphiphilic layer may be a monolayer or a bilayer.

The membrane 7 may be a lipid bilayer. Suitable lipid bilayers are disclosed in WO2008/102121, WO2009/077734 and WO2006/100484. Methods for forming lipid bilayers are known in the art. Lipid bilayers are commonly formed by the method of Montal and Mueller (Proc. Natl. Acad. Sci. USA., 1972; 69: 3561-3566).

The nanopore 8 may be any transmembrane pore. The nanopore 8 may be biological or artificial. Suitable nanopores 8 include, but are not limited to, protein pores, polynucleotide pores and solid-state pores. The nanopore 8 may be a DNA origami pore (Langecker et al., Science, 2012; 338: 932-936).

The transmembrane protein pore may comprise a barrel or channel through which the ions may flow. The barrel or channel of the transmembrane protein pore typically comprises amino acids that facilitate interaction with nucleotides, polynucleotides or nucleic acids.

Transmembrane protein pores for use in accordance with the invention can be derived from β-barrel pores or α-helix bundle pores. The transmembrane pore may be derived from or based on for example Msp, α-hemolysin (α-HL), lysenin, CsgG, ClyA, Sp1 and hemolytic protein fragaceatoxin C (FraC). The transmembrane protein pore can be derived from CsgG. Suitable pores derived from CsgG are disclosed in WO 2016/034591. The transmembrane pore may be derived from lysenin. Suitable pores derived from lysenin are disclosed in WO 2013/153359.

The analytes (including, e.g., proteins, peptides, small molecules, polypeptide, polynucleotides) may be present in an analyte. The analyte may be any suitable sample. The analyte may be a biological sample. Any embodiment of the methods described herein may be carried out *in vitro* on an analyte obtained from or extracted from any organism or microorganism. The organism or microorganism is typically archaean, prokaryotic or eukaryotic and typically belongs to one of the five kingdoms: plantae, animalia, fungi, monera and protista. Some methods described herein may be carried out *in vitro* on an analyte obtained from or extracted from any virus.

The analyte can be a fluid sample derived from any source such as biological, industrial or environmental.. The analyte can comprise a body fluid. The body fluid may be obtained from a human or animal. The human or animal may have, be suspected of having or be at risk of a disease. The analyte may be urine, lymph, saliva, mucus, seminal fluid or amniotic fluid, but can be whole blood, plasma or serum. Typically, the analyte is human in origin, but alternatively it may be from another mammal such as from commercially farmed animals such as horses, cattle, sheep or pigs or may alternatively be pets such as cats or dogs. Alternatively, an analyte can be of plant origin.

The analyte may be a non-biological sample. The non-biological sample can be a fluid sample. An ionic salt such as potassium chloride may be added to the sample to effect ion flow through the nanopore.

The polynucleotide may be single stranded or double stranded. At least a portion of the polynucleotide may be double stranded.

The polynucleotide can be a nucleic acid, such as deoxyribonucleic acid (DNA) or ribonucleic acid (RNA). The polynucleotide can comprise one strand of RNA hybridised to one strand of DNA. The polynucleotide may be any synthetic nucleic acid known in the art.,

The polynucleotide can be naturally occurring or artificial.

The method may involve measuring two, three, four or five or more characteristics of a polynucleotide. The one or more characteristics can be selected from (i) the length of the polynucleotide, (ii) the identity of the polynucleotide, (iii) the sequence of the polynucleotide, (iv) the secondary structure of the polynucleotide and (v) whether or not the polynucleotide is modified.

The nanopore may be part of an array comprising a plurality of nanopores such as disclosed in WO2014064443. The polynucleotides may be caused to translocate the array of nanopores and a parameter such as current measured over time from which the sequence of the polynucleotide may be determined, such as disclosed in WO 2013041878 and WO2014135838. Suitable electrical measurements, are described in Stoddart D et al., Proc Natl Acad Sci, 12;106(19):7702-7, Lieberman KR et al, J Am Chem Soc. 2010;132(50):17961-72, and International Application WO 2000/28312. Other types of measurements may be carried out for example optical measurements such as fluorescence measurements and FET measurements. Optical measurements and electrical measurements may be carried out simultaneously, see for example Heron AJ et al., J Am Chem Soc. 2009; 131(5): 1652-3).

The polynucleotide may be labelled, and measurements may be carried out to determine the polynucleotide sequence from measurement of the labels. The analyte may be an expandomer such as disclosed by WO2014/074922.

The secondary structure may be measured in a variety of ways. For instance, if the method involves an electrical measurement, the secondary structure may be measured using a change in dwell time or a change in ion current flowing through the pore. This allows regions of single-stranded and double-stranded polynucleotide to be distinguished.

The presence or absence of any modification may be measured. The method can comprises determining whether or not the polynucleotide is modified by methylation, by oxidation, by damage, with one or more proteins or with one or more labels, tags or spacers. Specific modifications will result in specific interactions with the pore which can be measured using the methods described below.

In some methods described herein, the method may involve further characterizing the target polynucleotide. As the target polynucleotide is contacted with the pore, one or more measurements which are indicative of one or more characteristics of the target polynucleotide are taken as the polynucleotide moves with respect to the pore.

The method may involve determining whether or not the polynucleotide is modified. The presence or absence of any modification may be measured. The method can comprises determining whether or not the polynucleotide is modified by methylation, by oxidation, by damage, with one or more proteins or with one or more labels, tags or spacers.

Also provided is an apparatus for characterising a target analyte, such as a target polynucleotide. The apparatus comprises a plurality of the pores as disclosed herein and a plurality of membranes. The plurality of pores can be present in the plurality of membranes. The number of pores and membranes can be equal. A single pore can be present in each membrane.

The apparatus for characterising target analytes, may comprise or an array of pores as disclosed herein, in a plurality of membranes.

The apparatus can further comprise instructions for carrying out the method. The apparatus may be any conventional apparatus for analyte analysis, such as an array or a chip. Any of the embodiments discussed above with reference to the methods are equally applicable to the apparatus of the invention. The apparatus may further comprise any of the features present in the kit as disclosed herein.

The apparatus can be set up to carry out a method as disclosed herein.

The apparatus can comprise: a nanopore sensing device 30 that is capable of supporting the plurality of pores and membranes and being operable to perform analyte characterisation using the pores and membranes; and at least one port for delivery of the material for performing the characterisation.

Alternatively, the apparatus can comprise: a nanopore sensing device 30 that is capable of supporting the plurality of pores and membranes being operable to perform analyte characterisation using the pores and membranes; and at least one reservoir for holding material for performing the characterisation.

The apparatus can comprise: a sensor device that is capable of supporting the membrane and plurality of pores and membranes and being operable to perform analyte characterising using the pores and membranes; at least one reservoir for holding material for performing the characterising; a fluidics system configured to controllably supply material from the at least one reservoir to the sensor device; and one or more containers for receiving respective samples, the fluidics system being configured to supply the analytes selectively from one or more containers to the sensor device.

The apparatus may be any of those described in WO 2009/077734, WO 2010/122293, WO 2011/067559 or WO 00/28312.

Control of the movement of an analyte with respect to the nanopore e.g. speed of translocation, rejection of the analyte etc, can be managed by the systems and methods disclosed in WO2016/059427. Rejection of an analyte by the nanopore sensor can comprise ejection of the analyte from the nanopore.

The features in description above and drawings are interchangeable and compatible in light of the teaching herein. The present invention has been described above purely by way of example, and modifications can be made within the scope of the invention, which is defined by the appended claims.

## Claims

1. A nanopore support structure (1) comprising:
a first wall layer (2) of photoresist material comprising walls (3) defining an array of wells (4);
a second overhang layer (5) of photoresist material extending from the walls (3) across each of the wells in the array (4), the second overhang layer (5) defining an array of apertures (6) configured to support a membrane (7) suitable for insertion of a nanopore (8); and
protrusions protruding laterally of the extent of the second overhang layer (5) of photoresist material,
wherein the protrusions include inner protrusions (52) protruding laterally of the extent of the second overhang layer (5) of photoresist material inside the respective array of wells (4).

2. A nanopore support structure (1) according to claim 1, wherein the protrusions include outer protrusions (51) protruding laterally of the extent of the second overhang layer (5) of photoresist material outside the respective wells (4) and/or wherein the nanopore support structure (1) comprises outer protrusions protruding (51) laterally of the extent of an outer surface of the first wall layer (2) of photoresist material between the wells (4) of the array of wells.

3. A nanopore support structure (1) according to claim 2, wherein, for each aperture (6), the outer protrusions (51) define a respective protrusion wall (101) at least partially surrounding and set back from the aperture (6).

4. A nanopore support structure (1) according to claim 3, wherein the protrusion wall (101) partially surrounds the aperture (6) and has gaps therein.

5. A nanopore support structure (1) according claim 3 or 4, wherein each protrusion wall (101) is configured to enable a meniscus to be formed across the respective aperture (6) such that the meniscus extends, at least in part, into the respective aperture (6).

6. A nanopore support structure (1) according to any of claims 3 to 5, comprising, for each aperture (6), a plurality of peripheral outer protrusions (51a) comprising an inner surface facing towards the aperture (6) that defines the protrusion wall (101), and an outer surface facing away from the aperture (6).

7. A nanopore support structure (1) according to any one of the preceding claims, wherein the protrusions (52) are arranged to increase retention of apolar medium by the second overhang layer (5) of photoresist material.

8. A nanopore support structure (1) according to any one of the preceding claims, wherein the protrusions (52) are arranged to increase rigidity of the second overhang layer (5) of photoresist material.

9. A nanopore support structure (1) according to any one of the preceding claims, wherein the wells (4) have respective bases (10).

10. A nanopore support structure (1) according to claim 9, wherein the first wall layer (2) of photoresist material further defines the bases (10).

11. A nanopore support structure (1) according to claim 9, wherein the nanopore support structure (1) further comprises a substrate (20), the first wall layer (2) of photoresist material being fixed to the substrate and the substrate (20) defining the bases (10) of the wells (4).

12. A nanopore support structure (1) according to any one of the preceding claims, wherein the second overhang layer (5) of photoresist material and the first wall layer (2) of photoresist material comprise cured, negative photoresist material.

13. A nanopore support structure (1) according to claim 12, further comprising barriers (70) disposed in the wells (4), the barriers (70) being capable of reducing scattering of electromagnetic radiation of a wavelength for curing the negative photoresist material, wherein the wells (4) have respective bases (10) and the barriers (70) extend from the bases (10) to the second overhang layer (5) of photoresist material, and the barriers (70) extend from the walls (3) inwardly into the wells (4).

14. A nanopore support structure (1) according to any one of the preceding claims, further comprising membranes (7) extending across respective apertures (6) and optionally also a nanopore (8) inserted in at least some of the membranes (7).

15. A nanopore sensing device comprising:
first and second chambers (31, 32);
a planar structure (40) comprising a nanopore support structure (1) according to any one of the preceding claims, the planar structure (1) being provided with plural fluidic passages (41) which extend between the first and second chambers (31, 32) and include respective wells (4) of the array of wells and apertures (6) of said nanopore support structure (1), the apertures (6) opening into the first chamber (31); and
electrodes (11) arranged to sense a fluidic electrical potential in respective passages (41) between the nanopores (8) and the second chamber (32).

## Patentansprüche

1. Nanoporen-Trägerstruktur (1), umfassend:
eine erste Wandschicht (2) aus Photolackmaterial, die Wände (3) umfasst, die eine Anordnung von Vertiefungen (4) definieren;
eine zweite Überhangschicht (5) aus Photolackmaterial, die sich von den Wänden (3) über jede der Vertiefungen im Array (4) erstreckt, wobei die zweite Überhangschicht (5) ein Array von Öffnungen (6) definiert, die so konfiguriert sind, dass sie eine Membran (7) tragen, die zum Einfügen einer Nanopore (8) geeignet ist; und
seitlich über die Ausdehnung der zweiten Überhangschicht (5) aus Photolackmaterial hinausragende Vorsprünge,
wobei die Vorsprünge innere Vorsprünge (52) umfassen, die seitlich von der Ausdehnung der zweiten Überhangschicht (5) aus Photolackmaterial innerhalb der jeweiligen Anordnung von Vertiefungen (4) hervorstehen.

2. Nanoporen-Trägerstruktur (1) nach Anspruch 1, wobei die Vorsprünge äußere Vorsprünge (51) umfassen, die seitlich von der Ausdehnung der zweiten Überhangschicht (5) aus Photolackmaterial außerhalb der jeweiligen Vertiefungen (4) hervorstehen, und/oder wobei die Nanoporen-Trägerstruktur (1) äußere Vorsprünge umfasst, die seitlich von der Erstreckung einer Außenfläche der ersten Wandschicht (2) aus Photolackmaterial zwischen den Vertiefungen (4) der Vertiefungsanordnung hervorstehen (51).

3. Nanoporen-Trägerstruktur (1) nach Anspruch 2, wobei die äußeren Vorsprünge (51) für jede Öffnung (6) eine jeweilige Vorsprungswand (101) definieren, die die Öffnung (6) zumindest teilweise umgibt und von ihr zurückversetzt ist.

4. Nanoporen-Trägerstruktur (1) nach Anspruch 3, wobei die Vorsprungswand (101) die Öffnung (6) teilweise umgibt und Lücken darin aufweist.

5. Nanoporen-Trägerstruktur (1) nach Anspruch 3 oder 4, wobei jede Vorsprungswand (101) so konfiguriert ist, dass sie die Bildung eines Meniskus über der jeweiligen Öffnung (6) ermöglicht, so dass sich der Meniskus zumindest teilweise in die jeweilige Öffnung (6) erstreckt.

6. Nanoporen-Trägerstruktur (1) nach einem der Ansprüche 3 bis 5, die für jede Öffnung (6) eine Vielzahl von peripheren äußeren Vorsprüngen (51a) umfasst, die eine der Öffnung (6) zugewandte Innenfläche, die die Vorsprungswand (101) definiert, und eine von der Öffnung (6) abgewandte Außenfläche umfassen.

7. Nanoporen-Trägerstruktur (1) nach einem der vorhergehenden Ansprüche, wobei die Vorsprünge (52) so angeordnet sind, dass sie die Retention eines unpolaren Mediums durch die zweite Überhangschicht (5) aus Photolackmaterial erhöhen.

8. Nanoporen-Trägerstruktur (1) nach einem der vorhergehenden Ansprüche, wobei die Vorsprünge (52) so angeordnet sind, dass sie die Steifigkeit der zweiten Überhangschicht (5) aus Fotolackmaterial erhöhen.

9. Nanoporen-Trägerstruktur (1) nach einem der vorhergehenden Ansprüche, wobei die Vertiefungen (4) jeweilige Böden (10) aufweisen.

10. Nanoporen-Trägerstruktur (1) nach Anspruch 9, wobei die erste Wandschicht (2) aus Photolackmaterial ferner die Basen (10) definiert.

11. Nanoporen-Trägerstruktur (1) nach Anspruch 9, wobei die Nanoporen-Trägerstruktur (1) ferner ein Substrat (20) umfasst, wobei die erste Wandschicht (2) aus Photolackmaterial an dem Substrat befestigt ist und das Substrat (20) die Basen (10) der Vertiefungen (4) definiert.

12. Nanoporen-Trägerstruktur (1) nach einem der vorhergehenden Ansprüche, wobei die zweite Überhangschicht (5) aus Fotolackmaterial und die erste Wandschicht (2) aus Fotolackmaterial ausgehärtetes, negatives Fotolackmaterial umfassen.

13. Nanoporen-Trägerstruktur (1) nach Anspruch 12, die ferner Barrieren (70) umfasst, die in den Vertiefungen (4) angeordnet sind, wobei die Barrieren (70) in der Lage sind, die Streuung elektromagnetischer Strahlung einer Wellenlänge zum Aushärten des negativen Fotolackmaterials zu verringern, wobei die Vertiefungen (4) jeweilige Basen (10) aufweisen und die Barrieren (70) sich von den Basen (10) bis zur zweiten Überhangschicht (5) aus Photolackmaterial erstrecken und die Barrieren (70) sich von den Wänden (3) nach innen in die Vertiefungen (4) erstrecken.

14. Nanoporen-Trägerstruktur (1) nach einem der vorhergehenden Ansprüche, die ferner Membranen (7) umfasst, die sich über die jeweiligen Öffnungen (6) erstrecken, und optional auch eine Nanopore (8), die in mindestens einige der Membranen (7) eingefügt ist.

15. Nanoporensensor, umfassend:
eine erste und eine zweite Kammer (31, 32);
eine planare Struktur (40), die eine Nanoporen-Trägerstruktur (1) gemäß einem der vorhergehenden Ansprüche umfasst, wobei die planare Struktur (1) mit mehreren Fluidkanälen (41) versehen ist, die sich zwischen der ersten und der zweiten Kammer (31, 32) erstrecken und jeweilige Vertiefungen (4) der Vertiefungsanordnung und Öffnungen (6) der Nanoporen-Trägerstruktur (1) umfassen, wobei die Öffnungen (6) in die erste Kammer (31) münden; und
Elektroden (11), die so angeordnet sind, dass sie ein fluidisches elektrisches Potenzial in jeweiligen Durchgängen (41) zwischen den Nanoporen (8) und der zweiten Kammer (32) erfassen.

## Revendications

1. Structure de support de nanopore (1) comprenant :
une première couche de paroi (2) de matériau de résine photosensible comprenant des parois (3) définissant un réseau de puits (4) ;
une seconde couche en surplomb (5) de matériau de résine photosensible s'étendant des parois (3) à travers chacun des puits dans le réseau (4), la seconde couche en surplomb (5) définissant un réseau d'ouvertures (6) configuré pour supporter une membrane (7) adaptée à l'insertion d'un nanopore (8) ; et
des saillies dépassant latéralement de l'étendue de la seconde couche en surplomb (5) de matériau de résine photosensible,
dans laquelle les saillies comportent des saillies intérieures (52) faisant saillie latéralement de l'étendue de la seconde couche en surplomb (5) de matériau de résine photosensible à l'intérieur du réseau de puits (4) respectif.

2. Structure de support de nanopore (1) selon la revendication 1, dans laquelle les saillies comportent des saillies extérieures (51) faisant saillie latéralement de l'étendue de la seconde couche en surplomb (5) de matériau de résine photosensible à l'extérieur des puits (4) respectifs et/ou dans laquelle la structure de support de nanopore (1) comprend des saillies extérieures faisant saillie (51) latéralement de l'étendue d'une surface extérieure de la première couche de paroi (2) de matériau de résine photosensible entre les puits (4) du réseau de puits.

3. Structure de support de nanopore (1) selon la revendication 2, dans laquelle, pour chaque ouverture (6), les saillies extérieures (51) définissent une paroi de saillie respective (101) entourant au moins partiellement l'ouverture (6), et étant située en retrait de celle-ci.

4. Structure de support de nanopore (1) selon la revendication 3, dans laquelle la paroi de saillie (101) entoure partiellement l'ouverture (6) et présente des espaces en son sein.

5. Structure de support de nanopore (1) selon la revendication 3 ou 4, dans laquelle chaque paroi de saillie (101) est configurée pour permettre la formation d'un ménisque à travers l'ouverture respective (6) de sorte que le ménisque s'étend, au moins en partie, dans l'ouverture respective (6).

6. Structure de support de nanopore (1) selon l'une quelconque des revendications 3 à 5, comprenant, pour chaque ouverture (6), une pluralité de saillies extérieures périphériques (51a) comprenant une surface intérieure tournée vers l'ouverture (6) qui définit la paroi de saillie (101), et une surface extérieure tournée à l'opposé de l'ouverture (6).

7. Structure de support de nanopore (1) selon l'une quelconque des revendications précédentes, dans laquelle les saillies (52) sont agencées pour augmenter la rétention du milieu apolaire par la seconde couche en surplomb (5) de matériau de résine photosensible.

8. Structure de support de nanopore (1) selon l'une quelconque des revendications précédentes, dans laquelle les saillies (52) sont agencées pour augmenter la rigidité de la seconde couche en surplomb (5) de matériau de résine photosensible.

9. Structure de support de nanopore (1) selon l'une quelconque des revendications précédentes, dans laquelle les puits (4) ont des bases respectives (10).

10. Structure de support de nanopore (1) selon la revendication 9, dans laquelle la première couche de paroi (2) de matériau de résine photosensible définit en outre les bases (10).

11. Structure de support de nanopore (1) selon la revendication 9, dans laquelle la structure de support de nanopore (1) comprend en outre un substrat (20), la première couche de paroi (2) de matériau de résine photosensible étant fixée au substrat et le substrat (20) définissant les bases (10) des puits (4).

12. Structure de support de nanopore (1) selon l'une quelconque des revendications précédentes, dans laquelle la seconde couche en surplomb (5) de matériau de résine photosensible et la première couche de paroi (2) de matériau de résine photosensible comprennent un matériau de résine photosensible négatif durci.

13. Structure de support de nanopore (1) selon la revendication 12, comprenant en outre des barrières (70) disposées dans les puits (4), les barrières (70) étant capables de réduire la diffusion du rayonnement électromagnétique d'une longueur d'onde pour durcir le matériau de résine photosensible négatif, dans laquelle les puits (4) présentent des bases respectives (10) et les barrières (70) s'étendent des bases (10) à la seconde couche en surplomb (5) de matériau de résine photosensible, et les barrières (70) s'étendent des parois (3) vers l'intérieur des puits (4).

14. Structure de support de nanopore (1) selon l'une quelconque des revendications précédentes, comprenant en outre des membranes (7) s'étendant à travers des ouvertures respectives (6) et éventuellement aussi un nanopore (8) inséré dans au moins certaines des membranes (7).

15. Dispositif de détection de nanopores comprenant :
des première et seconde chambres (31, 32) ;
une structure plane (40) comprenant une structure de support de nanopore (1) selon l'une quelconque des revendications précédentes, la structure plane (1) étant pourvue de plusieurs passages fluidiques (41) qui s'étendent entre les première et seconde chambres (31, 32) et comportent des puits respectifs (4) du réseau de puits et des ouvertures (6) de ladite structure de support de nanopore (1), les ouvertures (6) débouchant dans la première chambre (31) ; et
des électrodes (11) agencées pour détecter un potentiel électrique fluidique dans des passages respectifs (41) entre les nanopores (8) et la seconde chambre (32).
